# EUROPÄISCHE PATENTSCHRIFT

(11) **EP 4 231 962 B1**
(45) Veröffentlichungstag und Bekanntmachung des Hinweises auf die Patenterteilung: **09.04.2025**
(21) Anmeldenummer: 21793948.7
(22) Anmeldetag: 19.10.2021
(51) Int. Cl.: A61L 27/36, A61L 27/22, A61L 27/34, A61F 2/10

(54) **BESCHICHTETES HAAR UND DESSEN VERWENDUNG**
COATED HAIR AND USE THEREOF
CHEVEUX REVÊTUS ET LEUR UTILISATION

(30) Priorität: 20.10.2020 EP 20202744
(43) Veröffentlichungstag der Anmeldung: 30.08.2023
(73) Patentinhaber: Strauch, Georg, 68526 Ladenburg (DE)
(72) Erfinder: Strauch, Georg, 68526 Ladenburg (DE)
(74) Vertreter: Jacobi, Markus Alexander
(86) Internationale Anmeldenummer: PCT/EP2021/078898
(87) Internationale Veröffentlichungsnummer: WO 2022/084285

(56) Entgegenhaltungen:
- WO-A1-2004/024176
- WO-A1-2020/106075
- WO-A2-2007/062387
- FR-A1- 3 025 431
- SOMMER CHRISTOPH ET AL: "Microbial repellence properties of engineered spider silk coatings prevent biofilm formation of opportunistic bacterial strains", MRS COMMUNICATIONS, vol. 11, no. 3, 1 June 2021 (2021-06-01), US, pages 356 - 362, XP093245294, ISSN: 2159-6859, Retrieved from the Internet <URL:https://epo.summon.serialssolutions.com/2.0.0/link/0/eLvHCXMwpV3fS9xAEB78AVYfWmsVtbbue03NbXaTW5CCqGdFW8RqoU9hk0zg8MyF5ETuf_CP7swmd5xFpKXvu5NldrMz387MNwCB_Ox7f9wJuZZpkoa-kZgr8mltYpVRQaLR6tC66MGvI3V9Gl2dqouZon6X_D6JUDYlDkzaVIz2yyxv2vcQoq_J5kfG42wDR7jijedhkW5nw2T6Vz9-Th9d2NpHjnyXLJ3xwq42bSHN82KeGqvJOlb> DOI: 10.1557/s43579-021-00034-y

## Beschreibung

Die vorliegende Erfindung betrifft das Gebiet der Haartransplantation aus Eigenhaar, Fremdhaar oder Kunsthaar. Die vorliegende Erfindung beschreibt implantierbares Haar, welches zumindest an einem implantierbaren Teil mit einer direkt mit dem Haar in Kontakt kommenden Zusammensetzung beschichtet ist, die mindestens ein Seidenprotein enthält. Zudem ist die vorliegende Erfindung mit Verfahren zum Herstellen solchen implantierbaren Haars und seiner kosmetischen Verwendung befasst.

Seit Jahrtausenden werden Frauen und Männer mit dem Problem des Haarausfalls konfrontiert. Die Haare schwächen sich ab oder fallen aus. Es gibt zahlreiche Gründe dafür, dazu zählen insbesondere genetische Veranlagung, hormonelle Veränderungen, Stress, Umwelteinflüsse, Krankheitsverläufe, Chemotherapie, Haarausriss, chemische Behandlung der Haare, Kolorierung der Haare, altersbedingte Veränderungen, falsche oder unzureichende Ernährung, Unfälle und viele andere Ursachen.

Auch Unfälle wie Abschürfungen, Nekrosen und Verbrennungen, oftmals einhergehend mit Narbenbildung, führen oftmals zur belastenden Minderung von Behaarung an Kopf- oder Körperpartien. Gerade asymmetrische Haarverluste können entstellend wirken. Zudem kann auch zur Hervorhebung männlicher Körpermerkmale eine stärkere Behaarung, etwa im Bereich des Barts oder der Brust von Interesse sein. Dies kann etwa bei einer Geschlechtsumwandlung von einem Körper mit weiblichem Erscheinungsbild in einen Körper mit männlichem Erscheinungsbild von besonderem Interesse sein.

Die Behandlungen gegen Haarverlust, inklusive medizinischer und nicht medizinischer, wie kosmetischer Methoden, führen häufig zu unzureichenden Ergebnissen, sind nicht für alle Menschen anwendbar und/oder haben häufig Nebenwirkungen. Bestehende Behandlungen gegen Haarverlust erfüllen häufig nicht die hohen Ansprüche und Erwartungen der Menschen.

Zudem haben sie häufig Nachteile, die auf die physische und mentale Gesundheit der Menschen einwirken. Auch soziale Faktoren können den Leidensdruck der Menschen noch weiter verstärken. Der Haarverlust kann zu physiologischen und emotionalen Problemen führen, mit denen die Menschen nicht umgehen können. Diese können in der Summe zu einem emotionalen Trauma und darüber hinaus führen, was in einer Negativspirale mündet.

Durch eine Behandlung bzw. Lösung des Haarproblems, welche die Ansprüche und Erwartungen besser erfüllt, insbesondere hinsichtlich mehr Natürlichkeit, weniger Risiken und Nebenwirkungen, mehr Pflegekomfort und/oder gutem Preis-Leistungsverhältnis, würde die Lebensqualität der Menschen deutlich verbessert werden. Die Menschen würden sich gesünder bzw. weniger benachteiligt fühlen, sie würden ggf. mehr soziale Anerkennung bekommen und weniger auf den Haarverlust reduziert werden und sie würden weniger Stress und negative Gefühle haben. Dies kann sich positiv auf das Leben der betroffenen Menschen auswirken, auch in sozialer, emotionaler und finanzieller Hinsicht. Die Nachfrage nach einem derartigen Haar-Produkt für Mensch und ggf. Tier erscheint hoch.

Ein klassisches Mittel zur optischen Erreichung einer vollständigen Kopfbehaarung stellt der Einsatz von Perücken und Toupets dar. Das natürliche Aussehen und ein gewöhnlicher, flexibler Alltag sind jedoch auch mit Perücken und Toupets oftmals nicht erreichbar. Es gibt zwar in den letzten Jahren bereits Fortschritte bei deren Entwicklung und Qualität, doch kommen die Vorschläge mehr einem Kaschieren des Haarproblems gleich als einer echten Lösung. Denn der Rand des Netzes, an dem die Haare befestigt sind, ist mit den Fingern deutlich spürbar. Dies lässt sowohl die tragende Person von Perücken und Toupets, als auch andere Menschen, die ihre Haare berühren, nie vergessen, dass es sich um eine unnatürliche Lösung handelt. Zudem müssen Perücken und Toupets immer wieder abgenommen und erneut für den Einsatz aufbereitet werden. Daher sind Klebereste zu entfernen, der Haarersatz ist zu waschen, zu frisieren und für das Ankleben vorzubereiten. Menschen, die diese Mittel verwenden, müssen ihren Alltag entsprechend anpassen. Da die Haare bei Perücken und Toupets meistens durch Verknotung mit dem Netz befestigt werden, können sie an den stark beanspruchten Knotenpunkten nach einer gewissen Zeit abbrechen. Dies wird etwa durch Pflegerituale wie Haarewaschen und Kämmen verstärkt. Auch der Rand des Netzes verliert mit der Zeit an Stabilität, da er beim Lösen der Klebstoffe und der Reinigung beansprucht wird. Dadurch bilden sich ausgefranzte Stellen, an denen die Haare keinen Halt mehr finden.

Während des regelmäßigen Wechsels der Perücken und Toupets entstehen Zeiträume, in denen diese Menschen unpässlich sind. Ein spontanes Treffen unter Freunden oder Arbeitskollegen kann dann nicht immer wahrgenommen werden. Auch Reisen und sportliche Aktivitäten wie Schwimmen und andere sind nur in begrenztem Maße möglich, da Perücken nur für begrenzte Zeit ausreichenden Halt bieten. Darüber hinaus kann Schweiß nicht einfach verdunsten, was zu Juckreiz führt. Auch die Körperwärme kann nur bedingt entweichen, wodurch häufig Kopfschmerzen die Folge sein können.

Zudem können sich an Perücken und Toupets auch Bakterien und sonstige Keime einnisten, was gesundheitliche Nachteile und Geruchsbildungen mit sich bringen kann. Auch eine mögliche Reaktion auf Klebstoff, wie Hautirritation oder Allergie, können dazu führen, dass Perücken abgesetzt und nicht wieder befestigt werden. Wirtschaftlich betrachtet ist zudem die Haltbarkeit der Perücken trotz Pflege begrenzt, was das Leben auch in technischer und finanzieller Hinsicht einschränkt. Der Einsatz von Perücken und Toupets ist somit keine befriedigende Lösung für viele Menschen.

Es wurden weitere Mittel entwickelt, die darauf abzielen, Haarverlust zu mindern oder zu kaschieren. Dazu zählen insbesondere hormonelle Behandlungen durch Medikamente, Eigenhaarverpflanzungen, Implantate von Kunsthaar, punktuelle Tätowierung zur Kurzhaarsimulation und weitere Verfahren. Bei vielen Verfahren fehlt wissenschaftliche Evidenz der Wirksamkeit oder es treten erhebliche Nebenwirkungen auf, die das Wohlbefinden der behandelten Person negativ beeinträchtigen können. Von besonderem Interesse ist daher die Implantation von Haar. Diese Implantate sind dauerhaft und naturähnlich in der Haut (etwa der Kopf- oder Gesichtshaut) verankert.

So ist die Implantation von Eigenhaar mitsamt dessen Wurzeln dem Grunde nach eine technische Lösung, die viele Vorteile mit sich bringt. Ein solches Verfahren ist jedoch aufwendig und kostenintensiv. Zudem stehen zur Implantation nur so viele Haare zur Verfügung wie zuvor an einer anderen Stelle des Individuums, wie etwa dem Hinterkopf, entfernt wurden. Daher ist die zur Verfügung stehende Ressource an Eigenhaar signifikant begrenzt. Eine Vollglatze ist mittels Transplantation von Eigenhaar nicht ausreichend behandelbar. Nach der Verpflanzung erleiden die Haarwurzeln zudem einen Schock, wodurch implantierte Haare zunächst ausfallen.

Erst nach Monaten wachsen dort die Haare wieder nach, wobei ein gewisser Prozentsatz die Prozedur nicht übersteht und verloren geht. Das Ergebnis ist somit nicht vorhersagbar und kann die Erwartungen der Menschen nicht zuverlässig erfüllen. Auch eine bleibende, unerwünschte Narbe nach der Entnahme eigener Haarwurzeln am Hinterkopf ist je nach Methode möglich.

Ohne eine schützende Schicht kann Eigenhaar ohne Wurzeln jedoch nicht oder nur mit nicht zu vernachlässigbaren unerwünschten Nebenwirkungen implantiert werden, da es wie bei eingewachsenem Haar (häufig vorkommend bei gekräuseltem Haar) zu einer Immunreaktion und Entzündung führen kann.

Fremd- oder Kunsthaar, insbesondere ohne Haarwurzeln, stünde als (nahezu) unbegrenzte Ressource zur Verfügung.

Die Implantation von Kunsthaar kann häufig den Anspruch eines natürlichen Aussehens nicht erfüllen, da die Haare z.B. aus Polyester bestehen, das in seiner Optik und Textur dem natürlichen Haar nicht in einem gewünschten Grade gleichkommt. Auch erkennt der Körper des Empfängers Fremd- oder Kunsthaar häufig als Fremdkörper und reagiert entsprechend darauf. Es kann zu einer erheblichen, unerwünschten Immunreaktion kommen. Aufgrund der andauernden Immunreaktion in der Haut kann es zu Entzündungen und unter Umständen sogar zu Nekrosen kommen. Dadurch können die Nervenendigungen in der Haut geschädigt werden, was bis zum Gefühlsverlust an den betroffenen Stellen führen kann.

Um die Immunreaktion zu unterdrücken, wird implantierbares Kunsthaar mit chemischen Mitteln beschichtet, beispielsweise mit Silber-Salzen. Dies hat jedoch negative Auswirkungen auf das körperliche Wohl, da das Immunsystem aktiv gehemmt werden kann und dennoch weiter gegen die Kunsthaarimplantate gerichtete Immunreaktionen auftreten können. Sobald das schützende, optional immunsuppressive Mittel vom implantierten Haar weg diffundiert ist, und somit nicht mehr in hinreichender Konzentration vorliegt, werden die implantierten Kunst- oder Fremdhaare meist abgestoßen. Auch die Verankerung eines wurzellosen und damit glatten Kunst- oder Fremdhaares führt häufig zu einer unzureichenden Verankerung.

Es wurde daher versucht, mittels unterschiedlicher Verankerungen eine Befestigung von Haar in der Haut mechanisch zu verbessern. So werden in GB 1,504,258 hakenförmige Verankerungen beschrieben, während GB 2,006,018 schlaufenförmiger Verankerungen lehrt. WO 2011/064772 lehrt unterschiedliche Formen von Verankerungsstrukturen und zudem, dass auch mehrere Einzelhaare über eine gemeinsame Verankerungsstruktur in der Haut verankert werden können. Über mechanische Verankerung hinausgehend werden Strukturen beschrieben, die das Collagen-System ansprechen, wie in US 10,561,490 und WO 2020/180682. Die Verankerung wird jedoch weitgehend mechanisch über Oberflächenstrukturen des implantierten Teils erreicht.

Um eine Immunreaktion zu vermindern wurden inerte Beschichtungen der zu implantierenden Teile des Haars beschrieben, wie Beschichtungen mit Gold, siehe US 4,517,997. Im Fall einer inerten Beschichtung wird das Haar jedoch rein mechanisch in der Haut (etwa der Kopfhaut) gehalten und weder mit Nährstoffen versorgt werden noch anwachsen können. Der Stoffaustausch mit dem umgebenden Hautgewebe wird durch die Sperrschicht aus inertem Material vereitelt. Die Entfernung abgenutzten Haars muss über vergleichsweise harsche mechanische Verfahren erzielt werden, indem die künstliche Wurzel aus meist hartem inerten Material aus der Haut herausgerissen wird, was mit einer erheblichen unerwünschten Belastung einhergehen kann.

In US-A 2003/195625 wurde Haar nur vergleichsweise dünn mit einer Sperrschicht aus inertem Material, wie etwa Gold, beschichtet und zur mechanischen Verankerung mit einer verdickten Struktur aus bioresorbierbarem Material umgeben. Dadurch wird erreicht, dass das Haar nach einer gewissen Zeit, wenn das bioresorbierbare Material hinreichend abgebaut ist, gezielt wieder ausfällt. Hierdurch entfällt zwar die Notwendigkeit des harschen mechanischen Entfernens des abgenutzten Haars, jedoch wird keine dauerhafte Haarverankerung erzielt. Auch wird durch die Sperrschicht aus inertem Material die im implantierbaren Teil des Haars aufliegt, eine direkte Wechselwirkung und ein Stoffaustausch zwischen Haar und umgebenen Hautgewebe verhindert. Ein Einwachsen des Haars und eine Nährstoffaufnahme aus dem umgebenen Hautgewebe unterbleibt. Zudem ist die Verwendung zweier Schichten, einschließlich einer inerten Schicht aus Gold teuer und technisch nur mit erheblichem Mehraufwand möglich.

FR-A 3025431 beschreibt die Verwendung von Spinnenseidenfasern als künstliche Haar-ähnliche Struktur die keine Entzündungs- oder Immunreaktion hervorruft.

Es besteht daher ein Bedürfnis, ein Haarimplantat bereitzustellen, das gut verträglich im Hautgewebe und zugleich leicht herzustellen ist. Das Haarimplantat soll längerfristig in der Haut verbleiben können und dabei, nach einer gewissen Zeit, einen Stoffaustausch mit dem umgebenden Hautgewebe des Empfängers ermöglichen.

Überraschend wurde gefunden, dass durch eine Beschichtung mit einer Zusammensetzung, die ein oder mehrere Seidenproteine enthält, implantierbares Haar mit besonders guten Eigenschaften erhalten werden kann. Derartiges implantierbares Haar ist technisch einfach herzustellen. Es kann gut verträglich in der Haut verankert werden. Es kann auch längerfristig in der Haut verbleiben. Ein Stoffaustausch mit dem umgebenden Hautgewebe wird ermöglicht.

Insgesamt zeichnet sich insbesondere Spinnenseide durch ihre antibakterielle und antifungizide Wirkung aus. Sie ist wundheilungsfördernd, wasserfest, sehr hitzestabil und biologisch abbaubar. Die vorliegende Erfindung betrifft implantierbares Haar, das dadurch gekennzeichnet ist, dass es zumindest an einem implantierbaren Teil mit einer Zusammensetzung beschichtet ist, die mindestens ein Seidenprotein enthält.

Ein Aspekt der vorliegenden Erfindung betrifft implantierbares Haar, welches zumindest an einem implantierbaren Teil mit einer direkt mit dem Haar in Kontakt kommenden Zusammensetzung beschichtet ist, die mindestens ein Seidenprotein enthält.

Wie hierin verwendet ist der implantierbare Teil der Teil des Haars, der implantiert werden kann. Typischerweise erfolgt die Implantation in die Haut. Gemäß einer Ausführungsform erfolgt die Implantation in die Kopfhaut. Gemäß einer anderen Ausführungsform erfolgt die Implantation in eine oder mehrere andere Bereiche des Gesichts oder des menschlichen oder tierischen Körpers. So kann eine Implantation etwa im Bereich einer oder beider Augenbrauen, im Bereich einer oder beider Wimpern, im Bereich des Schnurrbarts, im Bereich des Kinnbarts, im Bereich des Backenbarts, im Bereich einer oder beider Koteletten, im Bereich der Brust, im Bereich eines oder beider Beine erfolgen. Die vorliegende Erfindung ist zur Neuerlangung oder Wiederherstellung der Behaarung (Haarwiederherstellung) geeignet.

Wie hierein verwendet, ist der Begriff "direkt mit dem Haar in Kontakt" im weitesten Sinne zu verstehen. So ist die Beschichtung bevorzugt nicht lediglich eine solche, die lediglich einen makroskopischen Stoff beschichtet, der wiederum mit dem Haar in Kontakt steht. Es wird verstanden werden, dass das Haar optional auch seinerseits beschichtet und/oder gefärbt und/oder anders verändert sein kann. Daher kann ein direktes in Kontakt kommen im Sinne der vorliegenden Erfindung auch mit beschichtetem Haar erfolgen. Es wird somit verstanden werden, dass optional zwischen dem Haar und der Zusammensetzung eine dünne Schicht (vorzugsweise nicht dicker als 0.5 mm, stärker bevorzugt nicht dicker als 0.1 mm, insbesondere nicht dicker als 0.05 mm) eines anderen Materials vorkommen kann. In diesem Fall wird der Fachmann auch dies als direkt mit dem (dann beschichteten) Haar in Kontakt kommend verstehen. Eine solche Zwischenbeschichtung kann optional die Anhaftung der Zusammensetzung und/oder des Seidenproteins an das Haar verbessern. Wenn eine solche optionale Zwischenbeschichtung vorliegt, ist diese bevorzugt bioresorbierbar (biologisch abbaubar). Wenn eine solche optionale Zwischenbeschichtung vorliegt, ist diese bevorzugt nicht inert. Bevorzugt stört oder verhindert eine optional vorhandene Zwischenbeschichtung nicht die dauerhafte Haarverankerung. Bevorzugt stört oder verhindert eine optional vorhandene Zwischenbeschichtung nicht den Stoffaustausch zwischen Haar und umgebenem Hautgewebe.

Die Zusammensetzung kann in bestimmten Ausführungsformen auch unmittelbar mit dem Haar in Kontakt kommen, ohne dass eine Zwischenschicht vorliegt.

Bevorzugt ist der Begriff "direkt mit dem Haar in Kontakt" dadurch charakterisiert, dass zwischen dem Haar und der Zusammensetzung keine Schicht vorliegt, die dicker als 0.5 mm, stärker bevorzugt nicht dicker als 0.1 mm, insbesondere nicht dicker als 0.05 mm ist.

In einer bevorzugten Ausführungsform ist der Begriff "direkt mit dem Haar in Kontakt" dadurch charakterisiert, dass zwischen dem Haar und der Zusammensetzung keine Schicht vorliegt, die biologisch inert ist, insbesondere wobei die Schicht nicht dicker als 0.5 mm, stärker bevorzugt nicht dicker als 0.1 mm, insbesondere nicht dicker als 0.05 mm ist.

In einer besonders bevorzugten Ausführungsform liegt keine Zwischenschicht vor. Daher ist besonders bevorzugt das Haar (daher dessen Keratinstruktur) in unmittelbarem Kontakt mit der Zusammensetzung, wie hierein definiert.

Die Beschichtung mit einer Zusammensetzung, die mindestens ein Seidenprotein enthält, kann auch als Schutzschicht verstanden werden. Dadurch wird eine besonders verträgliche Implantation in die Haut erreicht. Eine solche Beschichtung ermöglicht, insbesondere bei Implantation des implantierbaren Haars unter (weitgehend) sterilen Bedingungen, auch einen Schutz vor Bakterien und sonstigen Keimen, die auf oder in Haut und/oder Haar vorkommen können, sowie die Minderung von unerwünschten Immunreaktionen wie etwa Entzündungen.

Um die Funktion der Wundheilungsförderung durch die Spinnenseide zu nutzen, kann die Verankerung, die mindestens ein Seidenprotein enthält, zusätzlich eine leicht abtragbare und/oder bioresorbierbare äußere Schicht, die mindestens ein Seidenprotein enthält, haben. Nach der Implantation baut der Körper die leicht abtragbare und/oder bioresorbierbare Schicht schnell ab, transportiert sie zur Epidermis und sorgt dadurch für schnellere und infektionsarme Wundheilung. Hierbei ist "leicht abtragbar und/oder bioresorbierbar" im weitesten Sinne so zu verstehen, dass diese Schicht entfernt werden kann, insbesondere im Körper eines Individuums mechanisch und/oder biologisch abgetragen werden (auch: abgebaut werden). Sie kann dabei bioresorbiert werden. Dies kann optional eine Aufnahme in Gewebe und/oder Körperzellen und/oder extrazellulären Metabolismus sein. Auch können Abbauprodukte optional im Körper weiter metabolisiert und/oder aus dem Körper ausgeschieden werden.

Seidenprotein wird regelmäßig nicht als Fremdkörper wahrgenommen. Hierbei kann die Schutzschicht aus der Zusammensetzung das implantierbare Haar vom umgebenden Gewebe zumindest teilweise abtrennen, um eine Immunreaktion zu verringern oder zu verhindern. Auch an der Austrittstelle des implantierten Haars können durch die Schutzschicht aus der Zusammensetzung bakterielle Infektionen und Entzündungen vermindert oder verhindert werden. Es kann verhindert werden, dass Bakterien und sonstige Keime in die Stelle, in die das implantierbare Haar implantiert wird, eindringen oder sich an dem implantierbaren Haar festsetzen.

Im Gegensatz zu Perücken kann der Talg zudem von der Haut (etwa der Kopfhaut) auf natürlichem Weg auf die Haare (natürlich vorhandene und/oder implantierte Haare) übergehen und verfängt sich nicht im Netz von Perücken oder Toupets. Dies kann die Haare (natürlich vorhandene und/oder implantierte Haare) vor Umwelteinflüssen, physischen Einwirkungen und/oder Austrocknung schützen.

Die Haarstruktur kann stabilisiert werden. Für zusätzlichen Schutz kann implantierbares Haar nicht nur an der implantierten Stelle, sondern auch vollständig mit der Zusammensetzung, die mindestens ein Seidenprotein enthält, beschichtet werden. Dies kann zudem das Styling der Haare vereinfachen und verbessern.

Die Beschichtung des implantierbaren Teils des implantierbaren Haars mit der Zusammensetzung, die mindestens ein Seidenprotein enthält, kann den gesamten implantierbaren Teil umfassen oder einen Teil davon. Bevorzugt umfasst die Beschichtung des implantierbaren Teils des implantierbaren Haars mit der Zusammensetzung, die mindestens ein Seidenprotein enthält, mindestens 25%, mindestens 50%, mindestens 75%, mindestens 90% oder mindestens 95% der Oberfläche des implantierbaren Teils. Besonders bevorzugt umfasst die Beschichtung des implantierbaren Teils des implantierbaren Haars mit der Zusammensetzung, die mindestens ein Seidenprotein enthält, den gesamten implantierbaren Teil.

Optional kann die Beschichtung des implantierbaren Teils des implantierbaren Haars mit der Zusammensetzung, die mindestens ein Seidenprotein enthält, auch die Austrittsstelle des implantierbaren Haars aus der Haut des Empfängers nach der Implantation umfassen. Hierdurch kann die Austrittsstelle vor Bakterien und anderen Keimen geschützt werden und/oder auch hier eine Immunreaktion vermindert oder verhindert werden. Gemäß einer Ausführungsform der vorliegenden Erfindung umfasst die Beschichtung den implantierbaren Teil (etwa eine echte oder künstliche Haarwurzel und/oder einen echten oder künstlichen Haarfollikel und/oder eine echte oder künstliche Haarzwiebel) und die Austrittsstelle des implantierbaren Haars aus der Haut nach der Implantation.

Gemäß einer Ausführungsform der vorliegenden Erfindung umfasst die Zusammensetzung zu mindestens 10 Gew-%, zu mindestens 25 Gew-%, zu mindestens 50 Gew-%, zu mindestens 75 Gew-%, zu mindestens 80 Gew-%, zu mindestens 90 Gew-%, zu mindestens 95 Gew-% oder zu mindestens 99 Gew-%, ein oder mehrere Seidenproteine, bezogen auf die Trockenmasse der Zusammensetzung.

Gemäß einer Ausführungsform der vorliegenden Erfindung umfasst die Zusammensetzung zu mindestens 10 Gew-%, zu mindestens 25 Gew-%, zu mindestens 50 Gew-%, zu mindestens 75 Gew-%, zu mindestens 80 Gew-%, zu mindestens 90 Gew-%, zu mindestens 95 Gew-% oder zu mindestens 99 Gew-%, ein oder mehrere Seidenproteine, bezogen auf die Gesamtmasse der Zusammensetzung.

Die als Beschichtung verwendete Zusammensetzung, die mindestens ein Seidenprotein enthält, kann optional auch aus einem oder mehreren Seidenproteinen und optional dessen (kosmetisch verträglichen) Salzen bestehen.

Wie hierin verwendet, ist "kosmetisch verträglich" zu verstehen als akzeptabel bei der Implantation des implantierbaren Haars in der Haut.

Die als Beschichtung verwendete Zusammensetzung kann fest, pastös oder flüssig sein. Sie kann optional zusätzlich zu dem mindestens einen Seidenprotein ein kosmetisch verträgliches Lösungsmittel enthalten. Ein solches, kosmetisch verträgliches Lösungsmittel kann beispielsweise ausgewählt sein aus der Gruppe bestehend aus Wasser, einem kosmetisch verträglichen flüssigen Puffer, einem hydroalkoholischen Gemisch, enthaltend Wasser und bis zu 5 Vol.-% Ethanol, Dimethylsulfoxid (DMSO) und Kombinationen aus zwei oder mehr daraus.

Optional kann die als Beschichtung verwendete Zusammensetzung auch ein oder mehrere Konservierungsmittel, ein oder mehrere antibakterielle (etwa bakterizide und/oder bakteriostatische) Mittel, ein oder mehrere antifungizide Mittel, ein oder mehrere antivirale Mittel, blutungsstillende Mittel, ein oder mehrere Vitamine, ein oder mehrere, das Haarwachstum oder die Durchblutung fördernde bioaktive Stoffe, ein oder mehrere Lokalanästhetika, ein oder mehrere Verdickungsmittel, ein oder mehrere Hormone, ein oder mehrere Säureregulatoren oder eine Kombination aus zwei oder mehr der genannten Komponenten enthalten.

Optional kann die als Beschichtung verwendete Zusammensetzung, unabhängig von deren Inhaltstoffen, auch auf der Oberfläche des implantierbaren Haars eingetrocknet sein.

Optional kann die als Beschichtung verwendete Zusammensetzung auch zum Teil in das implantierbare Haar eindiffundiert sein. Bevorzugt verbleibt jedoch über 50 Gew. -% des mindestens einen Seidenproteins, bezogen auf die Gesamtmenge der Zusammensetzung, die auf oder an dem Haar verbleibt, auf der Oberfläche des implantierbaren Haars und weniger als 50 Gew-% des mindestens einen Seidenproteins, bezogen auf die Gesamtmenge in der Zusammensetzung, die auf oder an dem Haar verbleibt, diffundieren in das Haar ein.

Erfindungsgemäß steht das Haar zumindest teilweise in direktem Kontakt zu der Beschichtung. Daher grenzt die Beschichtung zumindest teilweise direkt an die von dieser Schicht umschlossene Haarstruktur an. Somit kann ein Stoffaustausch stattfinden.

Die Erfindung betrifft im Allgemeinen auch Haar-Implantate mit Seide (etwa Spinnenseide und/oder Raupenseide) mit medizinischer und ästhetischer Funktionalität.

Das Seidenprotein kann jedes bekannte Seidenprotein sein. Gemäß einer Ausführungsform weist das mindestens eine eingesetzte Seidenprotein mindestens 90% Sequenzhomologie oder mindestens 95% Sequenzhomologie oder mindestens 98% Sequenzhomologie zu oder Sequenzidentität mit einem Insekten-Seidenprotein oder einem Spinnen-Seidenprotein auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus der Familie der Echten Spinner *(Bombycidae),* der Webspinnen (Aranae), etwa der der Vogelspinnen *(Mygalomorphae),* der Echten Webspinnen (*Araneomorphae*) und/oder der Gliederspinnen *(Mesothelae)* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus einer der Gattungen *Bombyx* (z.B. *Bombyx mori, Bombyx mandarina), Araneus, Nephila, Antheraea* (z.B. *Antheraea pernyi, Antheraea yamamai, Antheraea mylitta, Antheraea roylei, Antheraea proyeli, Antheraea paphia, Antheraea frithi, Antheraea assama), Arachnura, Caerostris, Argiope, Cyrtophora, Celaenia, Gasteracantha, Ordgarius, Neoscona, Zygiella, Parawixia, Neoscona, Dolophones, Aculeperia, Eriophora, Anepsion, Tegenaria, Heurodes, Cyclosa, Astracantha, Eriovixia, Nephilengys, Herennia, Acusilas, Neoscona, Poltys, Arkys, Poecilopachys, Hyalophora* (z.B. *Hyalophora cecropia), Samia* (z.B. *Samia cynthia), Attacus (Attacus atlas), Circula* (z.B. *Circula trifenestrata), Gonometa* (z.B. *Gonometa postica, Gonometa rufobrunnea)* und/oder *Anaphe* (z.B. *Anaphe panda, Anaphe moloneyi)* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus einer der Gattungen *Bombyx, Araneus, Nephila, Antheraea, Arachnura, Caerostris, Argiope, Cyrtophora, Celaenia, Gasteracantha, Ordgarius, Neoscona, Zygiella, Parawixia, Neoscona, Dolophones, Aculeperia, Eriophora, Anepsion, Tegenaria, Heurodes, Cyclosa, Astracantha, Eriovixia, Nephilengys, Herennia, Acusilas, Neoscona, Poltys, Arkys, Poecilopachys* auf.

Gemäß einer Ausführungsform weist das Seidenprotein mindestens 90% Sequenzhomologie oder mindestens 95% Sequenzhomologie oder mindestens 98% Sequenzhomologie zu oder Sequenzidentität mit einem Seidenprotein einer Gattung ausgewählt aus der Gruppe bestehend aus *Bombyx, Araneus Antheraea* und *Nephila* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein einer Spezies ausgewählt aus der Gruppe bestehend aus *Bombyx mori, Bombyx mandarina, Antheraea pernyi, Antheraea yamamai, Antheraea mylitta, Antheraea roylei, Antheraea proyeli, Antheraea paphia, Antheraea frithi, Antheraea assama Araneus didenatus, Nephila clavipes, Araneus bicentenarius, Arachnura higginsi, Araneus circulissparsus, Araneus diadematus, Caerostris darwini, Argiope picta, Argiope trifasciata Nephila antipodiana, Cyrtophora beccarii, Celaenia excavata, Gasteracantha kuhlii, Argiope aurantia, Ordgarius furcatus, Ordgarius magnificus, Neoscona nautica, Neoscona rufofemorata, Zygiella calyptrata, Parawixia dehaani, Neoscona oxancensis, Gasteracantha cancriformis, Gasteracantha arcuata, Cyrtophora moluccensis, Cyrtophora parnasia, Dolophones conifera, Dolophones turrigera, Gasteracantha doriae, Gasteracantha mammosa, Cyrtophora exanthematica, Aculeperia ceropegia, Eriophora pustulosa, Anepsion depressium, Gasteracantha quadrispinosa, Eriophora transmarina, Araneus bicentenarius, Nephila maculata, Gasteracantha hasseltii, Tegenaria atrica, Heurodes turrita, Cyclosa insulana, Astracantha minax, Araneus mitificus, Eriovixia laglaisei, Cyclosa bifida, Nephilengys malabarensis, Argiope versicolor, Herennia ornatissima, Argiope aemula, Cyrtophora unicolor, Cyrtophora hirta, Argiope keyserlingi, Acusilas coccineus, Argiope argentata, Gasteracantha cancriformis, Neoscona domiciliorum, Argiope aetheria, Argiope Keyserlingi, Poltys illepidus, Arkys clavatus, Arkys lancearius, Poecilopachys australasia, Nephila clavipes, Nephila senegalensis, Nephila madagascariensis, Hyalophora cecropia, Samia Samia cynthia, Attacus atlas, Circula trifenestrata, Gonometa postica, Gonometa rufobrunnea, Anaphe panda* und/oder *Anaphe moloneyi* auf.

Gemäß einer Ausführungsform weist das Seidenprotein mindestens 90% Sequenzhomologie oder mindestens 95% Sequenzhomologie oder mindestens 98% Sequenzhomologie zu oder Sequenzidentität mit einem Seidenprotein einer Spezies ausgewählt aus der Gruppe bestehend aus *Bombyx mori, Antheraea pernyi, Araneus diadematus, Nephila clavipes, Araneus bicentenarius* und *Caerostris darwini* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus *Bombyx mori* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus *Antheraea pernyi* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus *Araneus diadematus* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus *Nephila clavipes* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus *Araneus bicentenarius* auf.

Gemäß einer Ausführungsform weist das mindestens eine Seidenprotein mindestens 90% Sequenzhomologie oder mindestens 95% Sequenzhomologie oder mindestens 98% Sequenzhomologie zu oder Sequenzidentität mit einem Seidenprotein aus *Caerostris darwini* auf.

Weitere gemäß der vorliegenden Erfindung einsetzbare Seidenproteine und deren biochemische Eigenschaften werden in WO 2007/025719, WO 2006/008163, WO 2004/024176, DE-A 10333253, US 2007/196429, US 2007/214520, US 2009/123967 und WO 2011/069643 beschrieben.

Gemäß einer Ausführungsform der vorliegenden Erfindung ist das mindestens eine Seidenprotein dadurch gekennzeichnet, dass es ausgewählt ist aus der Gruppe bestehend aus Fibroin, Sericin, Spidroin 1 und Spidroin 2. Spidroin 2 kann optional auch *"Araneus diadematus* Fibroin 3" (ADF-3) bzw. *Araneus diadematus* Fibroin 4" (ADF-4) sein wie etwa in WO 2007/025719 und WO 2006/008163 beschrieben. ADF-3 kann eine Sequenz aufweisen, die mindestens 90% Homologie oder mindestens 95% Homologie oder mindestens 98% Homologie oder mindestens 99% Homologie zu SEQ ID NO: 1 aus WO 2006/008163 aufweist. ADF-4 kann eine Sequenz aufweisen, die mindestens 90% Homologie oder mindestens 95% Homologie oder mindestens 98% Homologie oder mindestens 99% Homologie zu SEQ ID NO: 2 aus WO 2006/008163 aufweist. ADF-3 kann eine Sequenz wie in SEQ ID NO: 1 aus WO 2006/008163 aufweisen. ADF-4 kann eine Sequenz wie in SEQ ID NO: 2 aus WO 2006/008163 aufweisen.

Gemäß einer Ausführungsform der vorliegenden Erfindung umfasst das mindestens eine Seidenprotein mindestens zwei, mindestens drei, mindestens vier oder mehr als vier Sequenzabschnitte, die jeweils eine der folgenden Sequenzen SEQ ID NO: 1 bis 6 aufweisen:

| | |
|---|---|
| GPGXX | (SEQ ID NO: 1), |
| GVPGX | (SEQ ID NO: 2), |
| GSGXX | (SEQ ID NO: 3), |
| GGYXX | (SEQ ID NO: 4), |
| PQQXX | (SEQ ID NO: 5), |
| GYGXX | (SEQ ID NO: 6), |

wobei jedes X unabhängig voneinander jede beliebige natürliche Aminosäure sein kann, vorzugsweise ausgewählt ist aus der Gruppe bestehend aus V, G, A, S, Y, P und Q.

Gemäß einer Ausführungsform der vorliegenden Erfindung umfasst das mindestens eine Seidenprotein mindestens zwei, mindestens drei, mindestens vier oder mehr als vier gleiche Sequenzabschnitte einer Sequenz SEQ ID NO: 1 bis 6.

Fibroin ist ein allgemein bekanntes faserartiges Protein, das beispielhaft von den Spinndrüsen der Seidenraupe *(Bombyx mori),* aber auch anderen Spezies sezerniert wird. Beispielhaft kann Fibroin eine schwere Kette der folgenden Sequenz SEQ ID NO: 7 umfassen:

MRVKTFVILCCALQYVAYTNANINDFDEDYFGSDVTVQSSNTTDEIIRDASGAVIEEQITTK
KMQRKNKNHGILGKNEKMIKTFVITTDSDGNESIVEEDVLMKTLSDGTVAQSYVAADAGA
YSQSGPYVSNSGYSTHQGYTSDFSTSAAVGAGAGAGAAAGSGAGAGAGYGAASGAGA
GAGAGAGAGYGTGAGAGAGAGYGAGAGAGAGAGYGAGAGAGAGAGYGAGAGAGAG
AGYGAGAGAGAGAGYGAGAGAGAGAGYGAASGAGAGAGYGQGVGSGAASGAGAGA
GAGSAAGSGAGAGAGTGAGAGYGAGAGAGAGAGYGAASGTGAGYGAGAGAGYGGA
SGAGAGAGAGAGAGAGAGYGTGAGYGAGAGAGAGAGAGYGAGAGAGYGAGYGV
GAGAGYGAGYGAGAGSGAASGAGSGAGAGSGAGAGSGAGAGSGAGAGSGAGAGSG
AGAGSGAGAGSGAGAGSGTGAGSGAGAGYGAGAGAGYGAGAGSGAASGAGAGSGA
GAGSGAGAGSGAGAGSGAGAGSGAGAGYGAGAGAGYGAGAGAGYGAGAGVGYGAG
AGSGAASGAGAGSGAGAGSGAGAGSGAGAGSGAGAGSGAGAGSGAGAGSGAGAGS
GAGAGSGAGVGYGAGVGAGYGAGYGAGAGAGYGAGAGSGAASGAGAGAGAGAGTG
SSGFGPYVANGGYSRSDGYEYAWSSDFGTGSGAGAGSGAGAGSGAGAGSGAGAGSG
AGAGSGAGAGYGAGVGVGYGAGYGAGAGAGYGAGAGSGAASGAGAGSGAGAGSGA
GAGSGAGAGSGAGAGSGAGAGSGAGAGSGAGAGSGAGAGSGAGAGSGAGVGSGAG
AGSGAGAGVGYGAGAGVGYGAGAGSGAASGAGAGSGAGAGSGAGAGSGAGAGSGA
GAGSGAGAGSGAGAGSGAGAGSGAGAGSGAGVGYGAGVGAGYGAGYGAGAGAGYG
AGAGSGAASGAGAGSGAGAGSGAGAGSGAGAGSGAGAGSGAGAGSGAGAGSGAGA
GSGAGAGSGAGAGSGAGAGSGAGAGYGAGAGAGYGAGYGAGAGAGYGAGAGSGAA
SGAGSGAGAGSGAGAGAGSGAGAGSGAGAGSGAGAGSGAGAGSGAGAGSGAGAGY
GAGVGAGYGAGYGAGAGAGYGAGAGSGAASGAGAGSGAGAGSGAGAGSGAGAGSG
AGAGSGAGAGSGAGAGSGAGVGYGAGYGAGAGAGYGAGAGSGAASGAGAGAGAGA

GTGSSGFGPYVAHGGYSGYEYAWSSESDFGTGSGAGAGSGAGAGSGAGAGSGAGAG
SGAGYGAGVGAGYGAGYGAGAGAGYGAGAGSGAGSGAGAGSGAGAGSGAGAGSGA
GAGSGAGAGSGAGAGSGAGAGSGAGAGSGAGAGSGAGAGYGAGYGAGAGAGYGAGAGSGAG
SGAGAGSGAGAGSGAGAGSGAGAGSGAGAGSGAGAGSGAGAGSGAGAGYGAGVGA
GYGAGYGAGAGAGYGAGAGSGAGSGAGAGSGAGAGSGAGAGSGAGVGSGAGAGSG
AGAGSGAGAGSGAGAGYGAGYGAGAGAGYGAGAGSGAGSGAGAGSGAGAGSGAGA
GSGAGAGSGAGAGSGAGAGSGAGAGSGAGVGYGAGVGAGYGAGYGAGAGAGYGAG
AGSGAASGAGAGAGAGAGTGSSGFGPYVANGGYSGYEYAWSSESDFGTGSGAGAGS
GAGAGSGAGAGSGAGAGSGAGAGYGAGYGAGAGAGYGAGAGSGAGSGAGAGSGAG
AGSGAGAGSGAGAGSGAGAGSGAGAGSGAGAGSGAGSGSGAGAGSGAGAGSGAGA
GYGAGVGAGYGVGYGAGAGAGYGAGAGSGAASGAGAGAGAGAGTGSSGFGPYVAH
GGYSGYEYAWSSESDFGTGSGAGAGSGAGAGSGAGAGSGAGAGSGAGAGSGAGAG
SGAGAGYGAGVGAGYGAAYGAGAGAGYGAGAGSGAASGAGAGSGAGAGSGAGAGS
GAGAGSGAGAGSGAGAGSGAGAGSGAGAGSGAGAGSGAGAGSGAGAGYGAGAGAG
YGAGAGSGAGSGAGAGSGAGAGSGAGAGSGAGAGSGAGAGSGAGSGSGAGAGSGA
GAGSGAGAGYGAGVGAGYGAGYGAGAGAGYGAGAGSGAGSGAGAGSGAGAGYGAG
AGAGYGAGYGAGAGAGYGAGAGTGAGSGAGAGSGAGAGSGAGAGSGAGAGSGAGA
GSGAGAGSGAGSGSGAGAGSGAGAGSGAGAGSGAGAGSGAGAGSGAGAGYGAGAG
AGYGAGYGAGAGAGYGAGAGSGAGSGAGAGSGAGAGSGAGAGSGAGAGYGAGYGA
GAGSGAASGAGAGAGAGAGTGSSGFGPYVAHGGYSGYEYAWSSESDFGTGSGAGAG
SGAGAGAGAGSGAGAGYGAGVGAGYGAGYGAGAGAGYGAGAGSGTGSGAGAGS
GAGAGYGAGVGAGYGAGAGSGAAFGAGAGAGSGAGAGSGAGAGSGAGAGSGAG
AGSGAGAGYGAGYGAGVGAGYGAGAGSGAASGAGAGSGAGAGSGAGAGSGAGAGS
GAGAGSGAGAGYGAGVGAGYGAGYGAGAGAGYGAGAGSGAASGAGAGSGAGAGAG
SGAGAGSGAGAGSGAGAGSGAGSGAGAGSGAGAGSGAGAGYGAGAGSGAASGAGA
GAGAGAGTGSSGFGPYVANGGYSGYEYAWSSESDFGTGSGAGAGSGAGAGSGAGAG
SGAGAGSGAGAGYGAGVGAGYGAGYGAGAGAGYGAGAGSGAGSGAGAGSGAGAGS
GAGAGSGAGAGSGAGAGSGAGAGSGAGAGYGAGAGSGAASGAGAGSGAGAGSGAG
AGSGAGAGSGAGAGSGAGAGSGAGAGYGAGVGAGYGVGYGAGAGAGYGAGAGSGA
GSGAGAGSGAGAGSGAGAGSGAGAGSGAGSGAGAGSGAGAGSGAGAGSGAGSGAG
AGSGAGAGYGVGYGAGAGAGYGAGAGSGAGSGAGAGSGAGAGSGAGAGSGAGSGA
GAGSGAGAGSGAGAGSGAGAGYGAGVGAGYGVGYGAGAGAGYGAGAGSGAGSGAG
AGSGAGAGSGAGAGSGAGAGSGAGAGSGAGAGSGAGAGSGAGSGAGAGSGAGAGS
GAGAGSGAGAGSGAGSGAGAGSGAGAGSGAGAGSGAGAGYGAGVGAGYGVGYGAG
VGAGYGAGAGSGAASGAGAGSGAGAGAGSGAGAGSGAGAGSGAGAGSGAGAGSGA
GAGSGAGAGYGAGYGAGVGAGYGAGAGVGYGAGAGAGYGAGAGSGAASGAGAGAG
SGAGAGTGAGAGSGAGAGYGAGAGSGAASGAGAGAGAGAGTGSSGFGPYVANGGYS

GYEYAWSSESDFGTGSGAGAGSGAGAGSGAGAGSGAGAGSGAGAGYGAGVGAGYG
AGAGSGAGSGAGAGSGAGAGSGAGAGSGAGAGSGAGAGYGAGAGSGTGSGAGAGS
GAGAGSGAGAGSGAGAGSGAGAGSGAGAGSGVGAGYGVGYGAGAGAGYGVGYGAG
AGAGYGAGAGSGTGSGAGAGSGAGAGSGAGAGSGAGAGSGAGAGSGAGAGSGAGA
GYGAGVGAGYGVGYGAGAGAGYGAGAGSGAGSGAGAGSGAGAGSGAGAGSGAGAG
SGAGSGAGAGSGAGAGSGAGAGSGAGSGAGAGSGAGAGYGVGYGAGAGAGYGAGA
GSGAGSGAGAGSGAGAGSGAGAGSGAGSGAGAGSGAGAGSGAGAGSGAGAGYGAG
VGAGYGVGYGAGAGAGYGAGAGSGAGSGAGAGSGAGAGSGAGAGSGAGAGSGAGA
GSGAGAGSGAGAGSGAGSGAGAGSGAGAGSGAGAGSGAGAGYGAGVGAGYGVGYG
AGAGAGYGAGAGSGAASGAGAGAGAGTGSSGFGPYVANGGYSGYEYAWSSESDF
GTGSGAGAGSGAGAGSGAGAGYGAGYGAGVGAGYGAGAGVGYGAGAGAGYGAGAG
SGAASGAGAGAGAGAGSGAGAGSGAGAGAGSGAGAGYGAGYGIGVGAGYGAGAGV
GYGAGAGAGYGAGAGSGAASGAGAGSGAGAGSGAGAGSGAGAGSGAGAGSGAGAG
SGAGAGYGAGYGAGVGAGYGAGAGVGYGAGAGAGYGAGAGSGAASGAGAGAGA
GAGSGAGAGSGAGAGSGAGAGSGAGAGSGAGAGSGAGAGSGAGAGSGAGAGSGAG
AGYGAGVGAGYGAGYGGAGAGYGAGAGSGAASGAGAGSGAGAGSGAGAGSGAGAG
SGAGAGSGAGAGYGAGAGSGAASGAGAGAGAGTGSSGFGPYVNGGYSGYEYAW
SSESDFGTGSGAGAGSGAGAGSGAGAGYGAGVGAGYGAGYGAGAGAGYGAGAGSG
AASGAGAGSGAGAGSGAGAGSGAGAGSGAGSGAGAGSGAGAGSGAGAGSGAGAGS
GAGAGSGAGAGYGAGVGAGYGAGYGAGAGAGYGAGAGSGAASGAGAGSGAGAGAG
SGAGAGSGAGAGSGAGAGSGAGAGSGAGAGSGAGSGAGAGSGAGAGYGAGYGAGV
GAGYGAGAGVGYGAGAGAGYGAGAGSGAASGAGAGSGSGAGSGAGAGSGAGAGSG
AGAGAGSGAGAGSGAGAGSGAGAGYGAGYGAGAGSGAASGAGAGAGAGTGSSG
FGPYVANGGYSGYEYAWSSESDFGTGSGAGAGSGAGAGSGAGAGYGAGVGAGYGAG
YGAGAGAGYGAGAGSGAGSGAGAGSGAGAGSGAGAGSGAGAGSGAGAGSGAGAGS
GAGAGSGAGAGYGAGYGAGAGAGYGAGAGVGYGAGAGAGYGAGAGSGAGSGAGAG
SGSGAGAGSGSGAGSGAGAGSGAGAGSGAGAGSGAGAGSGAGAGSGAGAGSGAGA
GYGAGYGIGVGAGYGAGAGVGYGAGAGAGYGAGAGSGAASGAGAGSGAGAGSGAG
AGSGAGAGSGAGAGSGAGAGSGAGAGSGAGAGSGAGAGSGAGAGYGAGAGVGYGA
GAGSGAASGAGAGSGAGAGSGAGAGSGAGAGSGAGAGSGAGAGSGAGAGSGAGSG
AGAGSGAGAGYGAGYGAGVGAGYGAGAGYGAGYGVGAGAGYGAGAGSGAGSGAGA
GSGAGAGSGAGAGSGAGAGSGAGAGSGAGSGAGAGYGAGAGAGYGAGAGAGYGAG
AGSGAASGAGAGAGAGSGAGAGSGAGAGSGAGSGAGAGSGAGAGYGAGAGSGAAS
GAGAGSGAGAGAGAGAGAGSGAGAGSGAGAGYGAGAGSGAASGAGAGAGAGTGSS
GFGPYVANGGYSRREGYEYAWSSKSDFETGSGAASGAGAGAGSGAGAGSGAGAGSG
AGAGSGAGAGGSVSYGAGRGYGQGAGSAASSVSSASSRSYDYSRRNVRKNCGIPRRQ
LVVKFRALPCVNC

Optional kann Fibroin zudem eine leichte Kette der folgenden Sequenz SEQ ID NO: 8 umfassen:

MKPIFLVLLVATSAYAAPSVTINQYSDNEIPRDIDDGKASSVISRAWDYVDDTDKSIAILNV
QEILKDMASQGDYASQASAVAQTAGIIAHLSAGIPGDACAAANVINSYTDGVRSGNFAGF
RQSLGPFFGHVGQNLNLINQLVINPGQLRYSVGPALGCAGGGRIYDFEAAWDAILASSDS
SFLNEEYCIVKRLYNSRNSQSNNIAAYITAHLLPPVAQVFHQSAGSITDLLRGVGNGNDAT
GLVANAQRYIAQAASQVHV

Sericin ist ein wohlbekanntes Protein, das in seiner natürlichen Umgebung Fibroin-Stränge zusammenhalten kann. Sericin kann beispielhaft von den Spinndrüsen der Seidenraupe *(Bombyx mori),* aber auch anderen Spezies sezerniert werden. Sericin kann beispielhaft eine der folgenden Sequenzen SEQ ID NO: 9 bis 11 aufweisen:
Sericin 1 kann SEQ ID NO: 9 umfassen: 
MRFVLCCTLIALAALSVKAFGHHPGNRDTVEVKNRKYNAASSESSYLNKDNDSISAGAHR
AKSVEQSQDKSKYTSGPEGVSYSGRSQNYKDSKQAYADYHSDPNGGSASAGQSRDSS
LRERNVHYVSDGEAVAASSDARDENRSAQQNAQANWNADGSYGVSADRSGSASSRR
RQANYYSDKDITAASKDDSRADSSRRSNAYYNRDSDGSESAGLSDRSASSSKNDNVFV
YRTKDSIGGQAKSSRSSHSQESDAYYNSSPDGSYNAGTRDSSISNKKKASSTIYADKDQI
RAANDRSSSKQLKQSSAQISSGPEGTSVSSKDRQYSNDKRSKSDAYVGRDGTVAYSNK
DSEKTSRQSNTNYADQNSVRSDSAASDQTSKSYDRGYSDKNIVAHSSGSRGSQNQKSS
SYRADKDGFSSSTNTEKSKFSSSNSVVETSDGASASRESSAEDTKSSNSNVQSDEKSA
SQSSSSRSSQESASYSSSSSSSTLSEDSSEVDIDLGNLGWWWNSDNKVQRAAGGATK
SGASSSTQATTVSGADDSADSYTWWWNPRRSSSSSSSASSSSSGSNVGGSSQSSGSS
TSGSNARGHLGTVSSTGSTSNTDSSSKSAGSRTSGGSSTYGYSSSHRGGSVSSTGSSS
NTDSSTKNAGSSTSGGSSTYGYSSSHRGGSVSSTGSSSNTDSSTKSAGSSTSGGSSTY
GYSSRHRGGRVSSTGSSSTTDASSNSVGSSTSGGSSTYGYSSNSRDGSVSSTGSSSN
TDSNSNSAGSSTSGGSSTYGYSSNSRDGSVSSTGSSSNTDSNSNSAGSSTSGGSSTY
GYSSNSRDGSVSSTGSSSNTDASTDLTGSSTSGGSSTYGYSSDSRDGSVSSTGSSSNT
DASTDLAGSSTSGGSSTYGYSSDCGDGSVSSTGSSSNTDASTDLAGSSTSGGSSTYGY
SSDSRDGSVSSTGSSSNTDASTDLAGSSTSGGSSTYGYSSNSRDGSVSSTGSSSNTDA
STDLTGSSTSGGSSTYGYSSSNRDGSVLATGSSSNTDASTTEESTTSAGSSTEGYSSSS
HDGSVTSTDGSSTSGGASSSSASTAKSDAASSEDGFWWWNRRKSGSGHKSATVQSST
TDKTSTDSASSTDSTSSTSGASTTTSGSSSTSGGSSTSDASSTSSSVSRSHHSGVNRLL
HKPGQGKICLCFENIFDIPYHLRKNIGV
Sericin 2 kann SEQ ID NO: 10 umfassen: 
MKIPYVLLFLVGVAVVNALPNPLFGGLVKSLSKKKQIFEDKFENLKENVGEKFENLKENVG
EKVENLKENVGEKLENIKEKAGEKFENLKDNVGEKFENLKDNVGDKLEAAKEKAGEIKKK
LVDVGEDLKDELTEDKKIKISISKDEGLTLEKEGYKSDYDRNEYEERGSEHQEDNDSDGS
YSKGSEYEKYGEEEKYEERRTHDKFSIGKNGISAERTKSKRGERKEVEGEYEKDYERKE
NNGGSSEYSERERESLEKSKERYGEQSSKSFSLGKSGLKKQDNSKSYSDKEESKLEKE
KKYEKKTKINNERQLDEDENERRTVVGRDEQRQDDQSRDDQSRDDQSQDEETGSDDS
DKNRGKDTDDKYSETGTNKSSETKTGKRDGSKSGVTVEREKSESNKKSREFENKEAES
STYRDKNRSVNSGSERKSSGKDEEYSEQNSSNKSFNDGDASADYQTKSKKVEKNSAR
DKKEKEKTDTRNSDGTYKTSEREKEQSSRVNQSKGSNSRDSSESDKSGRKVNKETETY
SDKDAQTSESERTQSKDNGGLKEKSSQSKNYGRDEKYSEEKERSSTGKFGSNDSRAR
STKAEEEHVRKSQEETHSEQREKTRSDGVTKYNDGDEHFDSDDTEKTKPNGRSPSHKD
TEKAKPNDRSSSDKDTEKTFDKNIDNKRPKDGSSSDKNVEQERENYKSESSRNEFENQ
KSAHSRYEDNGGLKEKSSQSKNYGRDEKYSEEKERSSTGKSGSNDSRARSTKAEEEHV
RKSQEETHSEQRGRTRSDGATTSNDNDKQYDSDDKNNSSTKHKKTVMRSEQSDSSQN
ENSTSESKKFAKTDGSNKYEAESSSHKQQEARKQSNRVVEKSTDGDNEESYRSESSSS
SSSSSSSSRSSSSSTYTGSHDDSSEE
Sericin 3 kann SEQ ID NO: 11 umfassen:

MNCKVALFLIVAIVAVQALPCQESRPRRCGYRYGCGGGRGRGSGVRRLDSGADDNQG
RSCLVGGSAESNDEDSDDRLSVINNTEGHATSSSFDEQSSSARQSSSSYQSQSYNKDS
SSTNESSDGGSGSGRTGSAGENGENSDDSSGATKGNSSKSSSSSQGQSASSSSSDEK
SSQSSSNSSNNSKSSSQSSSSQNSSGSKGSGSEESSNGGSGSGRTGSAGGTDEDSDD
SSGATKGNSSKSSSSSQGQSASSSSSDENSSQSSSNSSNNSKSSSQSSSGQNSSGSK
GSGSEESSNGGSGSGRNGSVGGTDEDSDDSSGATKGNSSKSSSSSQGQSASSSSSD
EKSSQSSSNSSNNSKSSSQSSSGQNSSGSKGSGSEESSNGGSGSGRNGSAGGTDED
SDDSSGATKGNSSKSSSSSQGQSASSSSSDEKSSQSSSNSSNNSKSSSQSSSGQNSS
GSKGSGSEESSNGGSGSGRNGSAGGTDEDSDDSSGATKGNSSKSSSSSQGQSASSS
SSDEKSSQSSSNSSNNSKSSSQSSSGQNSSGSKGSGSEESSNGGSGSGRTGSAGETD
EDSDDSSGATKGNSSKSSSSSQGKSASSSSSDEKSSQSSSNSSNNSKSSSQSSSSNNS
SGSKGSGSEESSNGGSGSGRTGSAGGSDEDSDDSSGATKGNSSKSSSSSQGQSASS
SSSDEKSSQSNSNSSNNSKSSSQSSSSNNSSGSKGSGSEESSNGGSGSGRTGSAGGT
DEDSDDSSGATKGNSSKSSSSSQGKSASSSSSDEKSSQSSSNSSNNSKSSSQSSSSKN
SSGSKGSGSEESSNGGSGSGRTGSAGGTDEDSDDSSGATKGNSSKSSSSSQGKSASS
SSSDEKSSQSNSNSSNNSKSSSQSSSSKNSSGSKGSGSEESSNGGSGSGRTGSAGGT
DEDSDDSSGATKGNSSKSSSSSQGKSASSSSSNEKSSQSSSNSSNNSKSSSQSSSSKN
SSGSKGSGSSESGDKKSSSRGSSGDNSDDDQTDSARSNSKRSTSSDASTKKSSSRKS

SNHRSSRSQQAHSSSSKQAQSSSSQQAQNSRSQQAHSSRSQQAHSSSSKQAQSSSS
KQAQSSSSKQAQSSSSKQAQSSSSQQAQSSRSQQAHSSRSQQAHSSSSKQAQSSSSK
QAQSSSSKQAQSSSSKQAQSSSSQQAQSSRSQQAHSSRSETDSKSSNSGGHSNHSSR
TEQKSSAKAISSSEQSQNFSSSSQKSAEAADGSESTQSASEYSSSQSKASSSFSASSAS
ESSSLSSDVNFEEKTD

Spidroine sind Faserproteine der Seide von einigen Spinnen, wie beispielhaft *Nephila clavipes.* Spidroin 1 kann beispielhaft die folgende Sequenz SEQ ID NO: 12 umfassen:

QGAGAAAAAAGGAGQGGYGGLGGQGAGQGGYGGLGGQGAGQGAGAAAAAAAGGA
GQGGYGGLGSQGAGRGGQGAGAAAAAAGGAGQGGYGGLGSQGAGRGGLGGQGAG
AAAAAAAGGAGQGGYGGLGNQGAGRGGQGAAAAAAGGAGQGGYGGLGSQGAGRG
GLGGQGAGAAAAAAGGAGQGGYGGLGGQGAGQGGYGGLGSQGAGRGGLGGQGAG
AAAAAAAGGAGQGGLGGQGAGQGAGASAAAAGGAGQGGYGGLGSQGAGRGGEGAG
AAAAAAGGAGQGGYGGLGGQGAGQGGYGGLGSQGAGRGGLGGQGAGAAAAGGAG
QGGLGGQGAGQGAGAAAAAAGGAGQGGYGGLGSQGAGRGGLGGQGAGAVAAAAG
GAGQGGYGGLGSQGAGRGGQGAGAAAAAAGGAGQRGYGGLGNQGAGRGGLGGQG
AGAAAAAAAGGAGQGGYGGLGNQGAGRGGQGAAAAAGGAGQGGYGGLGSQGAGR
GGQGAGAAAAAAVGAGQEGIRGQGAGQGGYGGLGSQGSGRGGLGGQGAGAAAAAA
GGAGQGGLGGQGAGQGAGAAAAAAGGVRQGGYGGLGSQGAGRGGQGAGAAAAAA
GGAGQGGYGGLGGQGVGRGGLGGQGAGAAAAGGAGQGGYGGVGSGASAASAAASR
LSSPQASSRVSSAVSNLVASGPTNSAALSSTISNVVSQIGASNPGLSGCDVLIQALLEVVS
ALIQILGSSSIGQVNYGSAGQATQIVGQSVYQALG

Spidroin 2 kann beispielhaft die folgende Sequenz SEQ ID NO: 13 umfassen:

PGGYGPGQQGPGGYGPGQQGPSGPGSAAAAAAAAAGPGGYGPGQQGPGGYGPGQ
QGPGRYGPGQQGPSGPGSAAAAAAGSGQQGPGGYGPRQQGPGGYGQGQQGPSGP
GSAAAASAAASAESGQQGPGGYGPGQQGPGGYGPGQQGPGGYGPGQQGPSGPGSA
AAAAAASGPGQQGPGGYGPGQQGPGGYGPGQQGPSGPGSAAAAAAAASGPGQQG
PGGYGPGQQGPGGYGPGQQGLSGPGSAAAAAAAGPGQQGPGGYGPGQQGPSGPGS
AAAAAAAAAGPGGYGPGQQGPGGYGPGQQGPSGAGSAAAAAAAGPGQQGLGGYGP
GQQGPGGYGPGQQGPGGYGPGSASAAAAAAGPGQQGPGGYGPGQQGPSGPGSASA
AAAAAAGPGGYGPGQQGPGGYAPGQQGPSGPGSASAAAAAAAAGPGGYGPGQQG
PGGYAPGQQGPSGPGSAAAAAAAAAGPGGYGPAQQGPSGPGIAASAASAGPGGYGP
AQQGPAGYGPGSAVAASAGAGSAGYGPGSQASAAASRLASPDSGARVASAVSNLVSS
GPTSSAALSSVISNAVSQIGASNPGLSGCDVLIQALLEIVSACVTILSSSSIGQVNYGAASQ
FAQVVGQSVLSAF

Ein Seidenprotein und Zusammensetzungen, die ein solches oder eine Mischung an Seidenproteinen enthalten, sind optional kommerziell erhältlich, etwa von AMSilk GmbH (Am Klopferspitz 19 im IZB, 82152 Planegg/München, Deutschland).

Ein Seidenprotein kann natürlich gewonnen oder synthetisch hergestellt werden. Ein Seidenprotein kann dem Organismus seiner ursprünglichen genetischen Herkunft entnommen werden, somit etwa aus oder direkt von Spinnen oder Raupen, gewonnen werden. Es kann etwa einer Seidenspinne, beispielhaft wie oben genannt, entnommen werden. Die Gewinnung von natürlicher Spinnenseide ist technisch vergleichswiese schwierig und aufwändig. Daher kann ein Seidenprotein alternativ auch heterolog exprimiert werden. Um heterolog exprimiertes Seidenprotein herzustellen kann dem Organismus seiner ursprünglichen genetischen Herkunft (etwa einer Spinne oder einer Raupe) Desoxyribonukleinsäure (DNS oder auch DNA) entnommen und in einen Wirtsorganismus (z.B. Bakterien, Insektenzellen, Säugetierzellen, Pflanzenzellen, Pilzzellen (z.B. Hefezellen)) überführt werden. Hierbei können für den Wirtsorganismus erkennbare Promotor- und optional Verstärker- (Enhancer- )Sequenzen und/oder Stopp-Sequenzen hinzugefügt werden. Das mindestens eine Seidenprotein kann von den Zellen in Zellkultur hergestellt werden. Ein Seidenprotein kann optional in der Wirtszelle verbleiben, die dann in einem Batchverfahren geerntet wird oder von der Wirtszelle sezerniert werden. Derartige biotechnologische Verfahren sind dem Fachmann wohlbekannt. Eine heterologe Expression kann beispielsweise in einem Bakterium, wie etwa *Escherichia coli* erfolgen, wie in US-A 2007/196429, US-A 2007/214520, US-A 2009/123967 und WO 2011/069643 beschrieben.

Der mindestens eine, implantierbare Teil des Haars kann an einer beliebigen Stelle des Haars liegen. Gemäß einer Ausführungsform ist der implantierbare Teil ein implantierbares terminales Ende des Haars. So kann das Haar beispielsweise auf einer Länge von bis zu 1 µm, von 1 bis 10 µm, von 5 bis 50 µm, von 10 bis 100 µm, von 50 bis 500 µm, von 100 µm bis 1 mm, von 0.5 bis 5 mm oder von mehr als 5 mm mit der das mindestens eine Seidenprotein enthaltenden Zusammensetzung beschichtet und/oder implantierbar sein. Dann kann das Haar mit diesem terminalen Ende in die Haut, etwa die Kopfhaut, implantiert werden.

Auch ist es möglich, dass beide terminalen Enden des Haars implantiert werden.

Dann kann optional nach der Implantation ein gebildeter Ringschluss aufgeschnitten werden, indem das Haar optional durch einen Schnitt zwischen den beiden implantierten terminalen Enden geteilt wird.

Gemäß einer anderen Ausführungsform ist der implantierbare Teil ein implantierbarer Knick in der Mitte des Haars oder ein knickbarer Teil in der Mitte des Haars ist.

Wie hierin verstanden ist die "Mitte" des Haars im weitesten Sinne zu verstehen als ein Teil des Haars, der nicht an einem terminalen Ende des Haars liegt, daher nicht an der Wurzel oder der Haarspitze. Bevorzugt, liegt die Mitte des Haars über die Haarlänge im Bereich der inneren 8/10 der Länge des Haars, daher im Bereich, der nicht an den jeweils äußeren 10% der Haarlänge liegt. Optional kann die Mitte auch (ungefähr) im Bereich der mathematischen Mitte des Haars (von der Wurzel bis zur Haarspitze) liegen (+/- 20%, bevorzugt +/-10%, insbesondere +/- 5%).

So kann das Haar geknickt werden und einen Anschnitt im Knick oder knickbaren Teil enthalten, der beispielsweise auf einer Länge von bis zu 1 µm, von 1 bis 10 µm, von 5 bis 50 µm, von 10 bis 100 µm, von 50 bis 500 µm, von 100 µm bis 1 mm, von 0.5 bis 5 mm oder von mehr als 5 mm mit der das mindestens eine Seidenprotein enthaltenden Zusammensetzung beschichtet sein kann. Dann kann dieser Teil implantiert werden. Bevorzugt wird dann ein V-förmiger Knick mit der Spitze der V-Form in die Haut implantiert.

Das implantierbare Haar kann jedes beliebige Haar sein. Wie hierin verwendet ist der Begriff "Haar" im weitesten Sinne zu verstehen als eine faserförmige Struktur, die ein optisches Erscheinungsbild wie ein menschliches oder tierisches Haar aufweist, bevorzugt eines menschlichen Haars oder eines Säugetiers, insbesondere eines menschlichen Haars. Optional kann das implantierbare Haar ein Haarfollikel und/oder eine Haarwurzel und/oder eine Haarzwiebel aufweisen oder keinen Haarfollikel und keine Haarwurzel und keine Haarzwiebel aufweisen.

Das implantierbare Haar kann Eigenhaar, Fremdhaar oder Kunsthaar sein. Gemäß einer Ausführungsform der vorliegenden Erfindung ist das implantierbare Haar ein menschliches Haar. Dieses kann ein Eigenhaar der behandelten Person sein oder kann ein Fremdhaar einer menschlichen oder tierischen Spenderin oder eines menschlichen oder tierischen Spenders sein.

Gemäß einer Ausführungsform der vorliegenden Erfindung ist das implantierbare Haar ein menschliches Kopfhaar. Auch können alternativ andere Körperhaare, wie beispielsweise Brusthaare, Achselhaare, Schamhaare, Beinhaare, Barthaare und/oder Armhaare verwendet werden.

Optional kann das menschliche implantierbare Haar ein Haarfollikel und/oder eine Haarwurzel und/oder eine Haarzwiebel aufweisen oder keinen Haarfollikel und keine Haarwurzel und keine Haarzwiebel aufweisen. Gemäß einer Ausführungsform der vorliegenden Erfindung ist das implantierbare Haar ein abgeschnittenes menschliches Haar ohne Haarfollikel und ohne Haarwurzel und ohne Haarzwiebel ist. Hierbei kann beispielhaft Haar von einem Frisör verwendet werden.

Gemäß einer alternativen Ausführungsform der vorliegenden Erfindung ist das implantierbare Haar ein synthetisches Haar oder ein tierisches Haar. Derartige Haare sind im Stand der Technik beschrieben. Ein synthetisches Haar kann auch als Kunsthaar bezeichnet werden. Kunsthaar kann auch Seide enthalten oder aus Seide bestehen.

Implantierbares Haar kann jede beliebige Dicke aufweisen. Gemäß einer Ausführungsform weist das implantierbare Haar über die gesamte Länge einen mittleren Durchmesser von 0,02 bis 0,15 mm auf.

Auch kann dünneres oder dickeres Haar eingesetzt werden. Das Haar kann beispielsweise einen mittleren Durchmesser von 0,01 bis 0,3 mm, von 0,03 bis 0,1 mm, von 0,02 bis 0,15 mm, von 0,03 bis 0,1 mm, von 0,04 bis 0,09 mm, von 0,04 bis 0,08 mm, von 0,05 bis 0,07 mm oder von 0,09 bis 0,5 mm aufweisen.

Die Beschichtung des implantierbaren Haars mit der Zusammensetzung, die mindestens ein Seidenprotein enthält, kann unterschiedlich dick sein. Sie kann über größere Bereiche eine (weitgehend) homogene Dicke aufweisen oder unterschiedliche Dicken aufweisen. Bei einer gleichmäßigen Beschichtung kann die Beschichtungsdicke im Nanometerbereich, im Mikrometerbereich oder im Millimeterbereich liegen. Hierbei kann die Beschichtung beispielsweise eine mittlere Schichtdicke von 0,01 bis 0,3 mm, von 0,03 bis 0,1 mm, von 0,02 bis 0,15 mm, von 0,03 bis 0,1 mm, von 0,04 bis 0,09 mm, von 0,04 bis 0,08 mm, von 0,05 bis 0,07 mm, von 0,09 bis 0,5 mm, von 0,09 bis 1 mm oder von 0,5 bis 2 mm aufweisen.

Die Beschichtung kann auch ein Tropfen (etwa ein Tropfen an mindestens einem terminalen Ende des Haars oder ein Tropfen an einer geknickten oder knickbaren Stelle des Haars) oder eine beliebige andere Form sein.

Die Verankerung des Haars kann über beliebige dazu geeignete Mittel erfolgen. Gemäß einer Ausführungsform der vorliegenden Erfindung weist implantierbares Haar an dem implantierbaren Teil keine Verdickung auf, die einen maximalen Umfang von mehr als dem Zweifachen des mittleren Umfangs des Haars entspricht. Dies ermöglicht die Verwendung eines nicht weiter vorbereiteten abgeschnittenen Haars (beispielsweise abgeschnittenen menschlichen Haars) zur Implantation, das an einem dazu geeigneten implantierbaren Teil mit der Zusammensetzung, die mindestens ein Seidenprotein enthält, beschichtet wird. Hierdurch kann ein Haarimplantat erhalten werden, das beschichtet ist mit der Zusammensetzung, die mindestens ein Seidenprotein enthält, ohne künstlich nachgebildeter Haarwurzel und/oder nachgebildetem Haarfollikel und/oder nachgebildeter Haarzwiebel und somit ohne spezielle Verankerung.

Wie hierin verwendet ist der mittlere Umfang des Haares zu verstehen als das Mittel des Umfangs über die gesamte Haarlänge betrachtet. Üblicherweise ist ein Haar, insbesondere ein abgeschnittenes Haar, weitgehend zylindrisch, so dass der Mittlere Umfang nahezu über die gesamte Länge vorliegt.

Gemäß einer Ausführungsform der vorliegenden Erfindung weist implantierbares Haar an dem implantierbaren Teil eine künstliche Verdickung auf, die durch die Zusammensetzung (enthaltend mindestens ein Seidenprotein) gebildet wird, wobei die Verdickung einen maximalen Umfang um das Haar herum von mindestens dem Zweifachen des mittleren Umfangs des Haars entspricht.

So kann der Umfang der künstlichen Verdickung, die aus der Zusammensetzung gebildet wird, je nach Anwendungsgebiet angepasst werden. Beispielsweise kann er einen maximalen Umfang vom 2,1-fachen bis 50-fachen, vom 2,5-fachen bis 30-fachen, vom 3-fachen bis 20-fachen oder vom 4-fachen bis 10-fachen des mittleren Umfangs des Haars aufweisen. Eine solche Struktur ermöglicht eine feste Verankerung in der Haut. Hierbei kann die Zusammensetzung optional über die Zeit nach der Implantation in der Haut abgebaut werden. Zur Verbesserung der Verankerung des Implantats kann optional hierbei eine künstliche Form der Haarwurzel und/oder des Haarfollikels und/oder einer Haarzwiebel gebildet werden.

Die künstliche Form kann dabei optional von der natürlichen Form abweichen, sowie optional zusätzliche Strukturen aufweisen, welche die Verankerung verbessern können. Hierdurch kann ein Haarimplantat erhalten werden, das beschichtet ist mit der Zusammensetzung, die mindestens ein Seidenprotein enthält, mit künstlich nachgebildeter Haarwurzel und/oder nachgebildetem Haarfollikel und/oder nachgebildeter Haarzwiebel und somit mit spezieller Verankerung.

Um die Verankerung des implantierbaren Haars zu verbessern, kann das implantierbare Haar an dem implantierbaren Teil eine künstliche Verankerungsstruktur aufweisen.

Optional kann die Zusammensetzung, die mindestens ein Seidenprotein enthält, zum Formen einer Verankerung am Haarimplantat verwendet werden, um die Befestigung des Haarimplantats in der Haut zu ermöglichen oder zu verbessern. Die Verankerung kann dabei verschiedene Formen haben. Sie kann die Form einer Haarwurzel und/oder eines Haarfollikels und/oder einer Haarzwiebel haben, wobei das anatomisch Innere einer Haarwurzel und/oder eines Haarfollikels und/oder einer Haarzwiebel dabei nicht abgebildet wird. Sie kann der Form einer Haarwurzel und/oder eines Haarfollikels und/oder einer Haarzwiebel ähnlich sein.

Sie kann aber auch deutlich von der Form einer Haarwurzel und/oder eines Haarfollikels und/oder einer Haarzwiebel abweichen. Eine Verankerungsstruktur kann optional ausgewählt sein aus der Gruppe bestehend aus der Form eines Kegels, eines stumpfen Kegels, eines verdickten Zylinders, eines Pilzes, eines Tropfens, eines Ellipsoids, eines Quaders (daher eines Würfels oder eines gestreckten Rechtecks), einer Spiralform, einer Schraubenform, einer Ringform, einer Kugel, einer Wabenstruktur, einer teilweisen Umwicklung des Haars mit einer Faser oder einem Band und einer Kombination aus zwei oder mehr daraus und optional von der Haarstruktur und/oder der Verankerungsstruktur abstehende Fortsätze (einschließlich Widerhaken) aufweisen.

Gemäß einer Ausführungsform ist eine Verankerungsstruktur ausgewählt aus der Gruppe bestehend aus der Form eines Kegels, eines stumpfen Kegels, eines verdickten Zylinders, eines Pilzes, eines Tropfens, eines Ellipsoids, eines Quaders (daher eines Würfels oder eines gestreckten Rechtecks), einer Spiralform, einer Schraubenform, einer Ringform, einer Kugel, und einer Kombination aus zwei oder mehr daraus und optional von der Haarstruktur (optional und/oder der Verankerungsstruktur) abstehende Fortsätze (einschließlich Widerhaken) aufweisen.

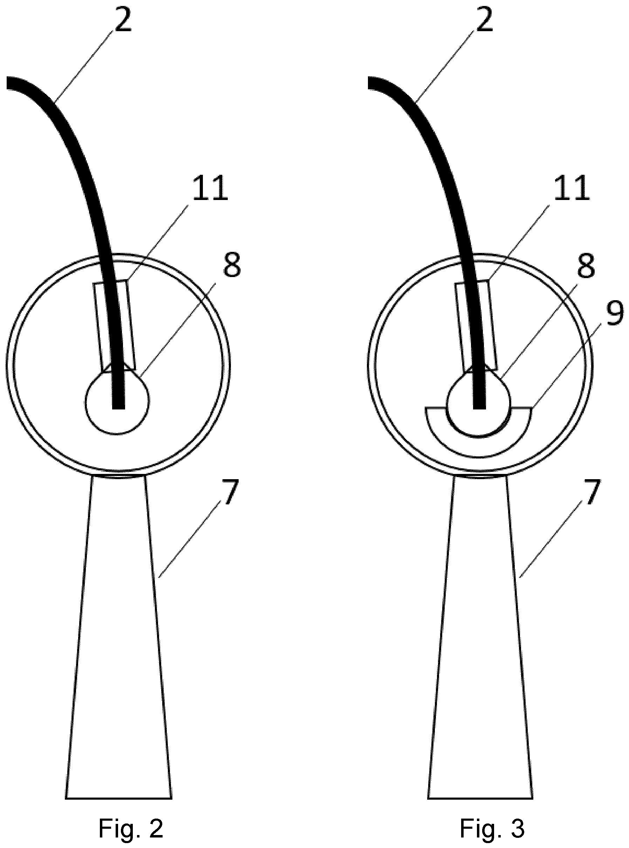

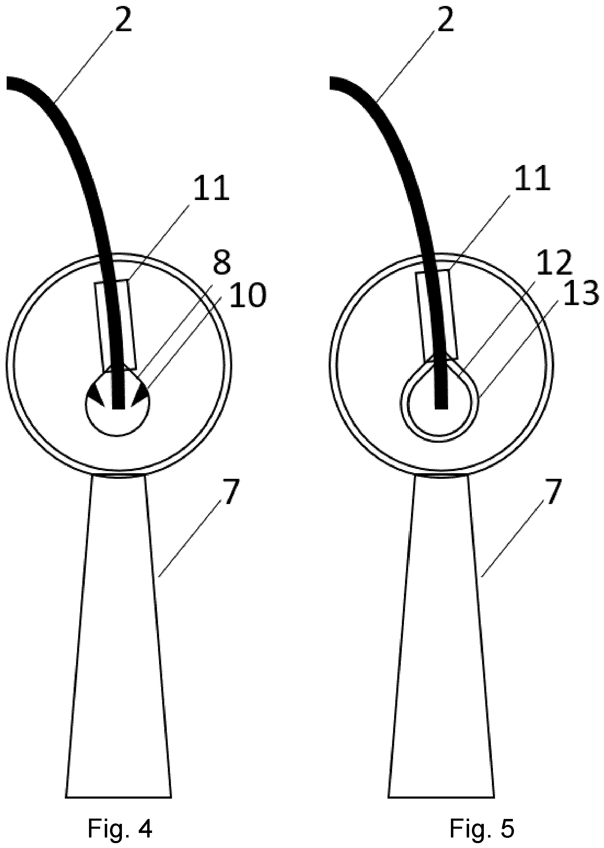

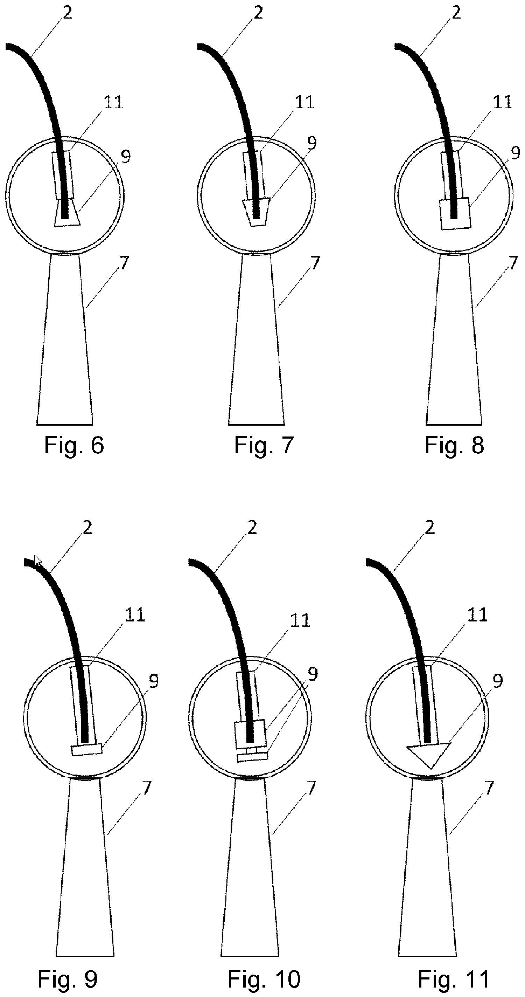

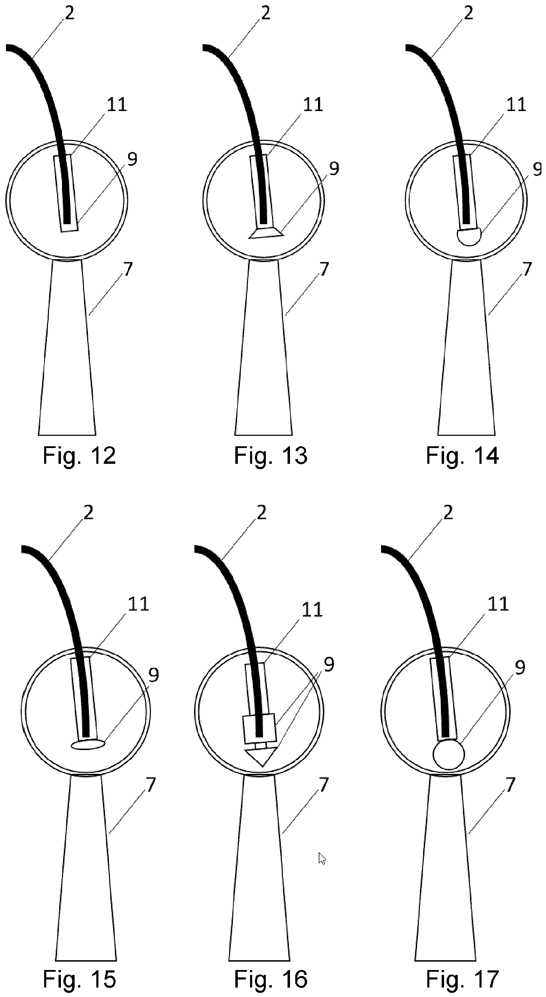

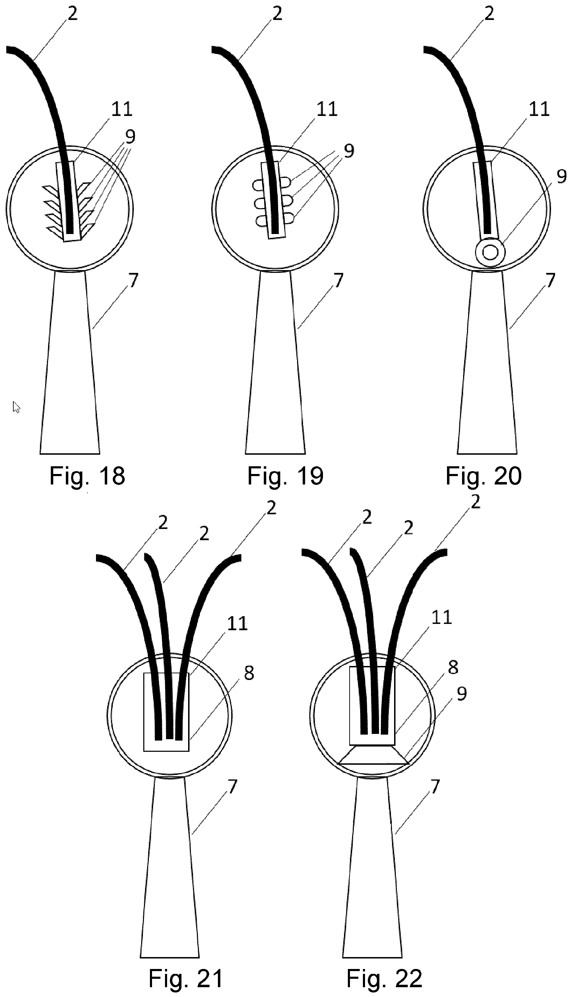

Konkrete Beispiele für Verankerungsstrukturen sind hierin beschrieben und exemplarisch in den Figuren 2 bis 27 dargestellt. Hierbei wird verstanden werden, dass die Strukturen vom Fachmann dreidimensional verstanden werden. Bevorzugt ist eine weitgehend symmetrische dreidimensionale Form anzunehmen.

Der Fachmann kennt die oben genannten Grundformen. Es wird zudem verstanden werden, dass eine Verankerungsstruktur insgesamt bevorzugt etwas breiter als die Dicke des Haars (oder der Summe der gemeinsam mit einer Verankerungsstruktur kombinierten Haare) am beschichteten Teil ist. In einer bevorzugten Ausführungsform weist die Verankerungsstruktur, ohne optionale abstehende Fortsätze, einen maximalen Durchmesser (in der Ebene des Durchmessers des beschichten Haars oder Haarbündels) auf, der 1,1 bis 20fach, insbesondere 1,2 bis 10fach, insbesondere 1,5 bis 5fach, so dick wie der mittlere Durchmesser des Haars (oder der Summe der gemeinsam mit einer Verankerungsstruktur kombinierten Haare) am beschichteten Teil ist. Dies ermöglicht eine gute Balance von einerseits einem effizienten und schonenden Implantieren des Haars und andererseits einer guten und vergleichsweise festen Verankerung in der Haut.

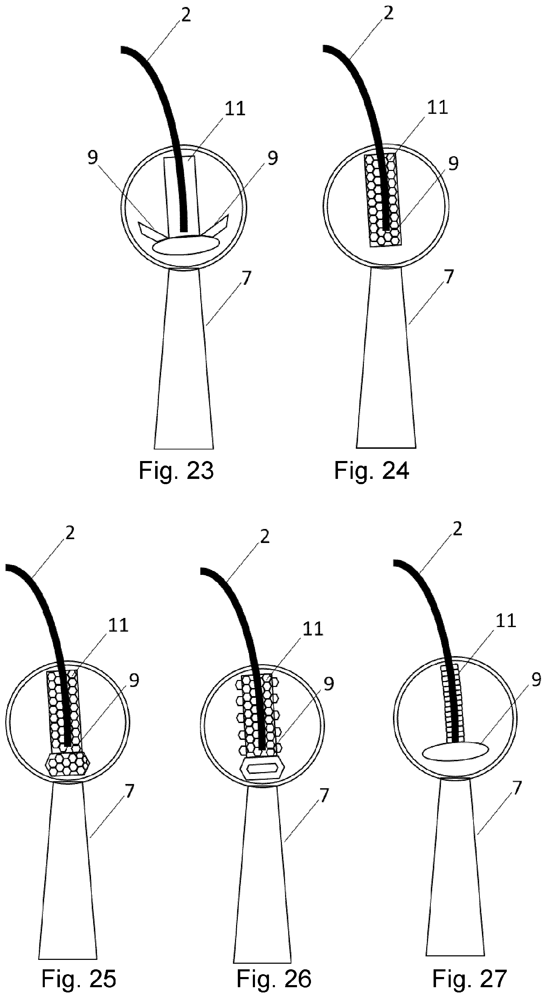

Eine Umwicklung des Haars mit einer Faser oder einem Band kann auf jede dazu geeignete Weise erfolgen. Eine Faser kann einen (weitgehend) runden oder (weitgehend) ovalen Querschnitt oder (weitgehend) quadratischen (auch: quaderförmigen) Querschnitt haben. Eine Faser kann aus (weitgehend) homogenem Material sein oder aber optional auch aus dünneren Fasern zusammengesetzt sein (z.B. geflochten, verdrillt oder parallelfaserig sein). Ein Band hat eine flache Geometrie. Ein Band kann aus (weitgehend) homogenem Material sein oder aber optional auch aus dünneren Fasern und/oder Bändern zusammengesetzt sein. Eine Faser oder ein Band kann in diesem Zusammenhang breit verstanden werden als eine Zusammensetzung gemäß der vorliegenden Erfindung umfassend oder aus einer solchen bestehend. Die Dicke einer Faser oder eines Bands kann frei gewählt werden. Eine dicke Faser oder ein dickes Band mit einem Durchmesser, der gleich dem Haar ist oder die Dicke des Haars übersteigt, kann optional verwendet werden. Dies ermöglicht eine schnelle Umwicklung. Eine dünne Faser oder ein dünnes Band mit einem Durchmesser der kleiner als der des Haars ist, kann optional verwendet werden. Dies ermöglicht eine (weitgehend) lückenlose Umwicklung und ermöglicht die Herstellung (besonders) wohldefinierter Formen. Bei der Umwicklung kann es sich um linienförmige (daher (weitgehend) parallele, spiralförmige) Wicklung, um netzartige Wicklung, um zopfartige Wicklung (kreuz und/oder quergewickelt) oder um eine Kombination aus verschiedenen Wicklungen handeln. Exemplarisch ist eine linienförmige (daher (weitgehend) parallele, spiralförmige) Wicklung in Figur 27 gezeigt, wobei die Ummantelung (11) eine spiralförmige Wicklung darstellt und in diesem Beispiel zusätzlich eine weitere Verankerungsstruktur (9) angebracht ist. Die Umwicklung kann optional direkt auf dem Haar erfolgen oder aber auf einer anderen Struktur, die dem Haar bereits aufliegt, insbesondere einer Struktur aus einer Zusammensetzung gemäß der vorliegenden Erfindung, die mindestens ein Seidenprotein enthält. Eine Umwicklung kann auf jede dazu geeignete Weise erfolgen. Beispielsweise kann das Haar (oder Haarbündel) (bevorzugt um seine (weitgehend) rotationssymmetrische Achse) gedreht werden und dabei eine oder mehrere Fasern und/oder ein oder mehrere Bänder aufwickeln. Alternativ kann auch das Haar (oder Haarbündel) unbewegt bleiben und die Umwicklung durch umlaufendes Material (z.B. eine umlaufende Spindel) erzielt werden. Optional kann das umwickelte Material mit dem Haar z.B. dessen Keratinstruktur) verbunden werden. Optional können verschiedene Abschnitte einer oder mehrerer Fasern und/oder eines oder mehrerer Bänder miteinander verbunden sein. Dies kann ein unerwünschtes Abwickeln verhindern. So kann beispielsweise eine spiralförmige Umwicklung zumindest an einem Ende verbundene Faser aufweisen, so dass das Abwickeln verhindert wird. Verfahren, die hierzu geeignet sind, sind unten weiter beschrieben.

Die Beschichtung, die mindestens ein Seidenprotein enthält, kann an der implantierten Stelle im Körper mit den Körperflüssigkeiten, wie etwa Wasser, unter Umständen interagieren. Dabei kann das Volumen der Beschichtung zunehmen, wodurch die Verankerung ermöglicht oder verstärkt werden kann. So kann, in einigen Ausführungsformen der vorliegenden Erfindung, die Beschichtung nach der Implantation Gewebsflüssigkeit aufnehmen und optional dadurch quellen. Hierdurch kann die Verankerung des Haars in der Haut verbessert werden.

Durch die erfindungsgemäßen Ausgestaltungen, wie hierin beschrieben, kann ein Haarimplantat erhalten werden, das beschichtet ist mit der Zusammensetzung, die mindestens ein Seidenprotein enthält, mit Verankerung, deren Form von der natürlichen Haarwurzel und/oder dem natürlichen Follikel und/oder der natürlichen Haarzwiebel abweicht. Die Verankerung kann dabei verschiedenste Formen haben wie vorstehend dargelegt. Die Verdickung kann somit verschiedene Formen sowie Einschnitte oder Ausstülpungen haben. Auch Widerhacken und Spiralformen sind zusätzlich oder alternativ möglich. Eine solche künstliche Befestigungsform kann optional so ausgestaltet sein, dass sie sich optional nach der Implantation des implantierbaren Haars ausbreiten und eine Befestigung oder Verbesserung der Befestigung des Haarimplantats ermöglichen kann. Zugleich kann die Zusammensetzung, die mindestens ein Seidenprotein enthält, auch in diesem Zusammenhang dem Schutz vor Bakterien und sonstigen Keimen und/oder der Minderung oder Verhinderung einer Immunreaktion des Körpers dienen. Die Verankerung des implantierbaren Haars kann auch eine oder mehrere Schleifen im Haar umfassen.

Eine künstliche Verankerungsstruktur kann auch aus der Zusammensetzung, die mindestens ein Seidenprotein enthält, aufgebaut sein.

Sie kann auch zusätzlich oder ausschließlich aus einem oder mehreren anderen Materialien aufgebaut sein und dann mit der Zusammensetzung, die mindestens ein Seidenprotein enthält, beschichtet sein. Derartige andere Materialien können etwa Kunststoffe sein, bevorzugt biologisch gut verträgliche Kunststoffe, wie beispielsweise Silikon, (Methyl)methacrylat, Polyvinylchlorid (PVC), Polymilchsäure oder andere verträgliche plastische Materialien oder Mischungen daraus. Auch können inerte Metalle wie etwa Gold, Platin oder Titan und deren Legierungen verwendet werden. Eine künstliche Verankerungsstruktur kann optional auch mit Graphen beschichtet sein. Eine künstliche Verankerungsstruktur ist bevorzugt mit einer ein Seidenprotein enthaltenden Zusammensetzung beschichtet.

Gemäß einer Ausführungsform der vorliegenden Erfindung ist das implantierbare Haar an dem implantierbaren Teil eingeschnitten. Auch dies ermöglicht eine Verankerung. So wird etwa das Einwachsen von Haut- und/oder Bindegewebe in die Einkerbungen der Struktur des implantierbaren Haars ermöglicht. Auch in eine schlaufenförmige Struktur des implantierbaren Haars kann optional Haut- und/oder Bindegewebe einwachsen.

Gemäß einer Ausführungsform der vorliegenden Erfindung weist das implantierbare Haar an dem implantierbaren Teil ein Haarfollikel oder eine Haarwurzel oder eine Haarzwiebel auf, die mit der Zusammensetzung beschichtet wird. Dies kann ein vollständiges und dauerhaftes Anwachsen des Haars (Eigenhaar oder Fremdhaar) ermöglichen. Somit kann das implantierte Haar von der Haut ernährt werden und unter Umständen sogar ein Längenwachstum zeigen. Die Beschichtung der Haarwurzel und/oder nachgebildetem Haarfollikel und/oder einer nachgebildeten Haarzwiebel mit der Zusammensetzung, die mindestens ein Seidenprotein enthält, kann auch in diesem Zusammenhang das Haarimplantat vor Bakterien und Keimen schützen und/oder eine Immunreaktion mindern oder verhindern.

Gemäß einer Ausführungsform der vorliegenden Erfindung sind die implantierbaren Haare so ausgestaltet, dass sie einzeln implantiert werden können. Daher weisen sie dann eine Beschichtungsstruktur auf, die meist ein einzelnes Haar enthält. Gemäß einer alternativen Ausführungsform der vorliegenden Erfindung sind zwei oder mehr implantierbare Haare an ihren implantierbaren Teilen in einer Beschichtungs- oder Verankerungsstruktur zusammengefasst. Hierdurch kann ein Haarimplantat erhalten werden, das beschichtet ist mit der Zusammensetzung, die mindestens ein Seidenprotein enthält, mit künstlich nachgebildeter Haarwurzel und/oder nachgebildetem Haarfollikel und/oder nachgebildeter Haarzwiebel und somit mit spezieller Verankerung, wobei es ein Haar oder mehrere Haare enthalten kann.

Die Beschichtung mit der Zusammensetzung, die mindestens ein Seidenprotein enthält, kann homogen sein. Alternativ kann die Beschichtung auch inhomogen sein, daher in unterschiedlichen Bereichen unterschiedlicher Festigkeit und/oder biologischer Stabilität aufweisen. Gemäß einer Ausführungsform der vorliegenden Erfindung weist die Beschichtung Zonen mit unterschiedlicher Festigkeit und/oder biologischer Stabilität auf. Gemäß einer Ausführungsform der vorliegenden Erfindung weist die Beschichtung im Inneren nahe am Haar eine höhere Festigkeit und/oder biologische Stabilität auf als an der Außenseite.

Dies kann ermöglichen, dass die äußere Schicht nach der Implantation des implantierbaren Haars schneller abgebaut wird und so ein Einwachsen des Haut- und/oder Bindegewebes gut ermöglicht wird, während der innere Teil das Haar lange schützt und einen langen Halt in der Haut ermöglicht. Hierdurch kann ein Haarimplantat erhalten werden, das beschichtet ist mit der Zusammensetzung, die mindestens ein Seidenprotein enthält, mit echter Haarwurzel und/oder echtem Haarfollikel und/oder echter Haarzwiebel, welches einem Menschen vom Kopfhaar oder Körperhaar entnommen wurde und im selben Menschen oder einem anderen Menschen wiedereingesetzt werden kann.

Beim Einsetzen des implantierbaren Haars in einem anderen Menschen handelt es sich somit um eine Transplantation von Fremdhaar. Dieses wird vom Immunsystem typischerweise als Fremdkörper erkannt und bekämpft werden. Die Immunreaktion kann durch die Beschichtung mit der Zusammensetzung, die mindestens ein Seidenprotein enthält, jedoch gemindert oder verhindert werden. Hierdurch kann eine Abstoßungsreaktion verhindert werden. Die Akzeptanz des Fremdhaars vom Empfänger und dessen Gewebe und Immunsystem kann verbessert werden.

Gemäß einer Ausführungsform der vorliegenden Erfindung ist die Beschichtung des implantierbaren Haars, umfassend das mindestens eine Seidenprotein, so gestaltet, dass nach einer Implantation das Seidenprotein oder ein Abbauprodukt auf das außerhalb des Körpers befindliche Haar und/oder die Kopfhaut abtransportiert wird und dort das Haar und/oder die Haut (etwa die Kopfhaut) pflegt.

Die Zusammensetzung, die mindestens ein Seidenprotein enthält, kann optional auch zum Beschichten von anderen Materialien eingesetzt werden, insbesondere Silikon oder Kunststoffen, an denen das Haar oder mehrere Haare für die Implantation (oder Transplantation) befestigt werden können.

Gemäß einer bevorzugten Ausführungsform wurde das implantierbare Haar mittels eines Verfahrens zur dreidimensionalen (3D-)Formgebung, insbesondere 3D-Druck, mit der Zusammensetzung beschichtet.

3D-Formgebung im Zusammenhang mit der vorliegenden Erfindung kann auch als 3D-Druck verstanden werden. 3D-Formgebungs-Verfahren (z.B. 3D-Druck), die für das Aufbringen (auch: Hinzufügen, Addieren) und/oder Abtragen (auch: Hinwegnehmen, Subtrahieren) von Zusammensetzungen geeignet sind, die mindestens ein Seidenprotein enthalten, sind dem Fachmann bekannt. Ein 3D-Formgebungs-Verfahren im Zusammenhang mit der vorliegenden Erfindung kann demnach ein subtraktives Verfahren (daher ein Abtragen von bereits beschichtetem Material) sein oder ein additives Verfahren (daher ein Hinzufügen von Material) sein oder eine Kombination aus beidem sein.

Gemäß einer bevorzugten Ausführungsform umfasst ein 3D-Formgebungs-Verfahren im Zusammenhang mit der vorliegenden Erfindung den Einsatz eines Lichtpulses, bevorzugt im ultravioletten (UV) Spektralbereich, besonders bevorzugt eines Femtosekunden-Lasers, insbesondere im UV-Bereich, wie beispielhaft in Sidhu et al. ("The processing and heterostructuring of silk with light", Nature Materials, 2017, DOI: 10.1038/NMAT4942, auch veröffentlicht als: Nature Materials, 16:938-945) beschrieben. Ein solches Verfahren ermöglicht sehr präzises Abtragen von Material (z.B. Seidenprotein), sehr präzise Schnitte in Material (z.B. Seidenprotein) und das Anhaften eines Materials (z.B. Seidenprotein) an ein anderes Material wie etwa Haar-Material (z.B. Keratin) und gleichartigen oder ähnlichen Materialien aneinander (z.B. eines Seidenproteins an gleichartiges oder verschiedenartiges Seidenprotein). Dadurch sind subtraktive und additive 3D-Formgebungs-Verfahren und Kombinationen daraus möglich.

Bei einem Femtosekunden-Laser werden Lichtpulse ausgesendet, deren Dauer im Bereich von wenigen Femtosekunden oder weniger (Femtosekunde:10⁻¹⁵ s) liegt. Das sich mit Lichtgeschwindigkeit ausbreitende Laserlicht legt in Vakuum und in Luft und ähnlichen Gasen bei atmosphärischem Druck innerhalb einer Femtosekunde lediglich eine Strecke von etwa 0,3 µm zurück, was etwa einem Hundertstel des Durchmessers eines menschlichen Haares entspricht. Ein technischer Vorteil eines Femtosekunden-Lasers kann daher die exakte und rückstandsfreie Abtragung kleinster Materialmengen ohne nennenswerte Wärmeübertragung (daher das Prinzip des Präzisionsskalpells) sein. Das Material kann von einem Femtosekunden-Laser auch direkt in Plasma umgewandelt werden, welches kaum thermisch mit dem Trägermaterial wechselwirkt.

Ein weiterer technischer Vorteil der Verwendung eines Femtosekunden-Lasers im Zusammenhang mit der vorliegenden Erfindung kann sein, dass eine Zusammensetzung, die mindestens ein Seidenprotein enthält, einem (optional auch schwächeren, daher. energieärmeren) Lichtpuls, bevorzugt ultravioletten (UV-)Lichts, ausgesetzt werden kann. Hierdurch kann die Zusammensetzung für einen sehr kurzen Moment ihre Eigenschaft ändern. Dabei kann eine feste oder pastöse Zusammensetzung für einen kurzen Moment etwa flüssig und dann wieder fest oder pastös werden. Wird die Zusammensetzung während so eines Lichtpulses mit einem Femtosekunden-Laser mit einem anderen Material in Kontakt gebracht (z.B. mit einem Haar oder einem Teil davon) so kann sie an diesem Material anhaften. Hierdurch kann eine bevorzugt dauerhafte Anhaftung der Zusammensetzung an das Haar oder einen Teil davon erreicht werden.

Das erfindungsgemäße Verfahren kann auch einen Verfahrensschritt der Bildung einer Verankerung der Zusammensetzung, die mindestens ein Seidenprotein enthält, am zu implantierenden Teil des Haars umfassen. Hierbei kann das Haar mit der Zusammensetzung, die mindestens ein Seidenprotein enthält, zunächst in Kontakt gebracht werden. Dann kann die Zusammensetzung an der Kontaktstelle mit dem Haar einem schwächeren Lichtpuls, etwa eines Femtosekunden-Lasers ausgesetzt werden. Dadurch kann erreicht werden, dass die Zusammensetzung an der Kontaktstelle mit dem Haar am Haar haften bleibt. Es kann sich somit eine Schicht mit der Zusammensetzung, die mindestens ein Seidenprotein enthält bilden.

Dieses Verfahren kann adäquat und analog auch angewendet werden, um weitere Schichten der Zusammensetzung, die mindestens ein Seidenprotein enthält, mit der Zusammensetzung zu verbinden. Die so gebildeten Schichten können auch unterschiedliche Festigkeiten haben.

Ein Haar oder ein Teil davon kann teilweise oder vollständig mit der Zusammensetzung, die mindestens ein Seidenprotein enthält, in Kontakt gebracht werden. Danach kann die Zusammensetzung einem (etwa schwächeren, daher energieärmeren) Lichtpuls des Femtosekunden-Lasers ausgesetzt werden. Dadurch kann die Zusammensetzung an der Kontaktstelle mit dem Haar anhaften. Mit diesem Verfahren lässt sich das Haar teilweise oder auch vollständig mit der Zusammensetzung verbinden, wodurch es teilweise oder auch vollständig von der Zusammensetzung ummantelt werden kann. Dies kann dazu beitragen, das Haar zu festigen, die Lebensdauer des Haars zu verlängern, es vor Umwelteinflüssen zu schützen und/oder das Styling des Haars zu verbessern.

Gemäß einer Ausführungsform der vorliegenden Erfindung kann der zu implantierende Teil des Haars mit einer oder mehreren Fasern und/oder einem oder mehreren Bändern, welche die Zusammensetzung der vorliegenden Erfindung enthalten oder daraus bestehen, in Kontakt gebracht werden. Dies kann, wie oben dargelegt, optional auch mittels Umwickeln geschehen, wie oben spezifischer ausgeführt. Optional kann die Umwicklung des Haars auch durchgeführt werden, während das Haar direkt oder indirekt mit einer flüssigen oder pastösen Zusammensetzung in Kontakt ist.

Optional kann ein umwickeltes Material mit dem Haar (z.B. dessen Keratinstruktur) verbunden werden. Dies kann auf jede beliebige Art erfolgen, wie beispielhaft durch einen Lichtpuls, insbesondere im UV-Bereich, wie hierin beschrieben. Besonders bevorzugt kann dies durch einen Femtosekunden-Laser erreicht werden, wie hierin beschrieben. Ein Lichtpuls, insbesondere im UV-Bereich, insbesondere ein Femtosekunden-Laser, wie hierin beschrieben, erlaubt zudem eine Nachbearbeitung einer Umwicklung. Dies kann beispielsweise auch eine Stabilisierung der Umwicklung durch (teilweises) Verbinden verschiedener Abschnitte der Fasern/Bänder sein.

Gemäß einer Ausführungsform der vorliegenden Erfindung kann der zu implantierende Teil des Haars mit der flüssigen oder pastösen Zusammensetzung, die mindestens ein Seidenprotein enthält, in Kontakt gebracht werden. Optional kann der zu implantierende Teil in eine solche Zusammensetzung etwa eingetaucht, damit bestrichen oder besprüht werden. Weitere Möglichkeiten sind hierin beschrieben. Ein Teil der Zusammensetzung kann dabei (beispielsweise in Tropfenform) am zu implantierenden Teil des Haars verbleiben. Danach kann die flüssige oder pastöse Zusammensetzung am zu implantierenden Teil des Haars trocknen und/oder aushärten. Aushärten ist hierin im weitesten Sinne so zu verstehen, dass die Festigkeit zunimmt. Dadurch bildet die Zusammensetzung bevorzugt eine Verankerung, die fest genug ist, um implantiert werden zu können. Hierbei reicht der Härtegrad bevorzugt aus, um im umliegenden Gewebe des Körpers, insbesondere der Haut, eine gewünschte Halbwertszeit zu erreichen. Die Zusammensetzung bleibt dabei bevorzugt jedoch elastisch. Elastizität der Verankerungsstruktur kann vorteilhaft sein, um nach der Implantation nicht oder weniger wahrgenommen zu werden und keine oder eine geringere Druckstelle und/oder keinen oder einen geringeren Schmerz zu erzeugen. Um das Aushärten zu beschleunigen, kann der zu implantierende Teil nach dem Inkontaktbringen mit der flüssigen oder pastösen Zusammensetzung auch an der Luft trocknen, alternativ auch durch Veränderung der Umgebung, beispielsweise unter Zuhilfenahme einer Belüftung oder durch Temperaturveränderung. Danach kann die gewünschte Struktur der Haarverankerung aus der gehärteten Zusammensetzung durch einen Laser, beispielsweise einem Femtosekunden-Laser, ausgeschnitten und/oder abgetragen werden.

Gemäß einer bevorzugten Ausführungsform umfasst die Herstellung des implantierbaren Haars im Zusammenhang mit der vorliegenden Erfindung das zumindest teilweise Entfernen von Seidenprotein. Gemäß einer bevorzugten Ausführungsform umfasst eine 3D-Formgebung im Zusammenhang mit der vorliegenden Erfindung das zumindest teilweise Entfernen von Seidenprotein mittels eines Lichtpulses, bevorzugt im ultravioletten (UV) Spektralbereich. Hierbei kann zunutze gemacht werden, dass Seidenproteine und optional auch andere Komponenten dieser Zusammensetzung im UV-Bereich absorbieren können. Gemäß einer bevorzugten Ausführungsform umfasst eine 3D-Formgebung im Zusammenhang mit der vorliegenden Erfindung das zumindest teilweise Entfernen von Seidenprotein mittels einer Plasma-Ablation. Gemäß einer bevorzugten Ausführungsform umfasst eine 3D-Formgebung im Zusammenhang mit der vorliegenden Erfindung das zumindest teilweise Entfernen von Seidenprotein mittels eines Femtosekunden-Lasers, insbesondere im ultravioletten (UV) Spektralbereich. Ein Verfahren basierend auf Plasma-Ablation mittels eines Femtosekunden-Lasers, insbesondere im ultravioletten (UV) Spektralbereich, ist dem Fachmann grundsätzlich bekannt (Sidhu et al., Nature Materials, 2017, DOI: 10.1038/NMAT4942, auch veröffentlicht als: Nature Materials, 16:938-945). Bevorzugt erfolgt das Abtragen mittels eines kurzzeitigen Lichtpulses von einer Dauer von <100 ps (Picosekunden) oder <10 ps oder <1 ps oder<100 fs (Femtosekunden) oder <10 fs. Bei einem Femtosekunden-Laser können daher durch stärkere Lichtpulse, insbesondere im UV-Licht-Bereich, kleinste Materialmengen an der flüssigen oder pastösen oder festen Zusammensetzung, die mindestens ein Seidenprotein enthält, abgetragen werden.

Durch die präzisen Anwendungsmöglichkeiten eines Femtosekunden-Lasers können verschiedenste Formen der Verankerung am Haar gebildet werden. Es können beispielsweise Wabenstrukturen gebildet werden, die Hohlräume enthalten können. Weitere Beispiele sind in den Figuren exemplarisch dargestellt. Dabei entstehende Hohlräume und/oder Kanten können mit flüssiger oder pastöser Zusammensetzung, die mindestens ein Seidenprotein enthält, in Kontakt gebracht werden. Dann kann die Zusammensetzung einem Lichtpuls des Femtosekunden-Lasers ausgesetzt werden, um eine Haftung der Zusammensetzung mit der Wabenstruktur zu verbessen oder zu ermöglichen.

Gemäß einer bevorzugten Ausführungsform umfasst die Herstellung des implantierbaren Haars das zumindest teilweise (etwa thermische) Aufschmelzen der Zusammensetzung und/oder des mindestens einen Seidenproteins, insbesondere mittels eines Femtosekunden-Lasers, insbesondere im ultravioletten (UV) Spektralbereich.

Aufschmelzen bedeutet im weitesten Sinne, dass die Zusammensetzung zumindest teilweise verflüssigt wird oder zumindest die Viskosität verringert wird. Ein Aufschmelzen ist ein Aufschmelzen durch, meist kurzfristige, meist lokale, Temperaturerhöhung. Bevorzugt wird die Temperaturerhöhung durch die Photonen-Einstrahlung bewirkt. Ein Aufschmelzen ist bevorzugt ein Aufschmelzen lediglich eines Teils der Zusammensetzung und/oder eines Seidenproteins mittels eines Lichtpulses, insbesondere eines UV-Lichtpulses.

Alternativ zu einem UV-Lichtpuls kann auch eine hohe Strahlungsintensität eines längerwelligen Lichts (mit dem nach energieärmeren Photonen) verwendet werden. Hierbei können Mehrphotonen-effekte (z.B. 2-Photonen- und/oder 3-Photonen-Effekte) genutzt werden, wie in Sidhu et al. (Nature Materials, 2017, DOI: 10.1038/NMAT4942, auch veröffentlicht als: Nature Materials, 16:938-945) beschrieben.

Nach dem kurzzeitigen Aufschmelzen härtet die Struktur bevorzugt wieder aus. Bevorzugt erfolgt das Aufschmelzen mittels eines kurzzeitigen Lichtpulses von einer Dauer von <100 ps oder <10 ps oder <1 ps oder<100 fs oder <10 fs. Bevorzugt erfolgt das Aufschmelzen und Aushärten eines Teils der Zusammensetzung und/oder des Seidenproteins sehr kurzzeitig, daher im Bereich von <100 ns (Nanosekunden) oder <10 ns oder <1 ns oder <100 ps oder <10 ps oder <1 ps oder<100 fs oder <10 fs. Dies kann eine Formgebung der 3D-Oberflächenstruktur im Nano- und/oder Mikrometerbereich bewirken, indem kleinste Tröpfchen des zumindest teilweise aufgeschmolzenen Materials an das Haar oder eine bereits ausgehärtete Struktur definiert anhaften.

Gemäß einer bevorzugten Ausführungsform umfasst das 3D-Formgebungs-Verfahren das teilweise Entfernen von Seidenprotein, das auf das Haar aufgebracht worden ist, insbesondere mittels Plasma-Ablation, insbesondere mittels eines Femtosekunden-Lasers, insbesondere im ultravioletten (UV) Spektralbereich.

Verfahren, die im Zusammenhang mit der vorliegenden Erfindung verwendet werden können, können auch dazu verwendet werden, mehrere Haare (etwa auch hunderte oder tausende) gleichzeitig zu bearbeiten und mit der Zusammensetzung, die mindestens ein Seidenprotein enthält, zu beschichten. Beschichtungsverfahren können automatisiert sein.

So können auch die beschriebenen 3D-Formgebungs-Verfahren dazu verwendet werden, mehrere Haare (etwa auch hunderte oder tausende) gleichzeitig zu bearbeiten und mit der Zusammensetzung, die mindestens ein Seidenprotein enthält, zu beschichten. Auch hierzu kann ein Lichtpuls (z.B. ein UV-Lichtpuls), etwa ein Femtosekunden-Laser eingesetzt werden. Dazu können mehrere Haare mit ihren zu implantierenden Teilen mit der Zusammensetzung, die mindestens ein Seidenprotein enthält, in Kontakt gebracht werden (z.B. durch Eintauchen in eine flüssige oder pastöse Zusammensetzung, etwa eine dünne Schicht in einer Petrischale). Danach kann eine festere Viskosität der Zusammensetzung erzielt werden (z.B. durch Belüftung, Temperaturveränderung oder Lichtveränderung, beispielsweise Einstrahlen von UV-Licht). Sobald die Zusammensetzung fest genug ist, um ihre Form zu bewahren und am Haar gut haftet, können die Haare mit ihren Haarverankerungen mit Hilfe eines Femtosekunden-Lasers herausgelöst werden. Dabei können kurze Lichtpulse eingesetzt werden, um die Verankerungsform für jedes Haar zu formen und das Haar dann zusammen mit der gebildeten Verankerung aus der Zusammensetzung zu lösen. Alternativ oder zusätzlich kann die Haarverankerung danach noch weiter mit dem Femtosekunden-Laser zu einer speziellen Verankerungsform gebildet werden, beispielsweise um eine Kegelform zu erzielen, Fortsätze zu formen, Ringform und viele andere. Ein Femtosekunden-Laser kann zur Bildung verschiedenster Verankerungsformen eingesetzt werden, dabei können die Formen auch miteinander kombiniert werden, beispielsweise auch eine Wabenform, Ringform mit Fortsätzen usw. Strukturbeispiele sind hierin beschrieben und auch exemplarisch in den Figuren 2 bis 27 gezeigt.

Gemäß einer bevorzugten Ausführungsform ist das implantierbare Haar zumindest teilweise mit einer festen oder pastösen Zusammensetzung beschichtet, die eine 3D-Oberflächenstruktur im Nano- oder Mikrometerbereich aufweist, die von der weitgehend zylindrischen Form (daher einer dem Haar ähnlichen Form, und bevorzugt auch einer Tropfenform) abweicht. Mögliche Formgebungen sind hierin und beispielhaft in den Figuren 2 bis 27 beschrieben.

Das implantierbare Haar gemäß der vorliegenden Erfindung kann auf jede dazu geeignete Weise hergestellt werden. Verfahren wie hierin beschrieben stellen bevorzugte Ausführungsformen dar.

Ein weiterer Aspekt der vorliegenden Erfindung betrifft auch ein Verfahren zum Herstellen implantierbaren Haars gemäß der vorliegenden Erfindung, wobei das Verfahren die folgenden Schritte umfasst:
(i) Bereitstellen von:
   (A) Haar, das sich nicht am menschlichen oder tierischen Körper befindet, und
   (B) einer Zusammensetzung, die mindestens ein Seidenprotein enthält; und
(ii) Inkontaktbringen zumindest eines implantierbaren Teils des Haars mit der Zusammensetzung.

Entsprechende Beispiele für Verfahren sind bereits oben ausgeführt, weitere Beispiele werden im Folgenden, sowie im Beispielteil weiter ausgeführt.

Es wird verstanden werden, dass alle im Zusammenhang mit dem implantierbaren Haar gemäß der vorliegenden Erfindung gemachten Definitionen und Ausführungsformen in entsprechender Weise auch für das Verfahren zum Herstellen eines solchen implantierbaren Haars gelten.

Gemäß einer bevorzugten Ausführungsform betrifft die vorliegende Erfindung ein Verfahren zum Herstellen implantierbaren Haars gemäß der vorliegenden Erfindung, wobei das Verfahren die folgenden Schritte umfasst:
(i) Bereitstellen von:
   (A) Haar, dass sich nicht am menschlichen oder tierischen Körper befindet, und
   (B) einer flüssigen oder pastösen oder festen (bevorzugt flüssigen oder pastösen) Zusammensetzung, die mindestens ein Seidenprotein enthält;
(ii) Inkontaktbringen zumindest eines implantierbaren Teils des Haars mit der flüssigen oder pastösen oder festen Zusammensetzung; und
(iii) optional Trocknen des aus Schritt (ii) erhaltenen implantierbaren Haars.

Die Schritte (ii) und (iii) können optional zwei oder mehr als zweimal wiederholt werden, was eine höhere Schichtdicke und/oder die Erzielung beliebiger Formen an Beschichtung ermöglichen kann.

Die vorliegende Erfindung betrifft auch implantierbares Haar, das nach einem Verfahren wie hierin beschrieben erhältlich ist oder erhalten wird.

Das in Schritt (i) des Bereitstellens der Ausgangsmaterialien eingesetzte Haar kann jedes Haar sein. Es kann etwa ein menschliches Haar sein, ein tierisches Haar sein oder ein synthetisches Haar sein. Entsprechende Ausführungsformen sind im Zusammenhang mit dem implantierbaren Haar näher erläutert.

Die in Schritt (i) des Bereitstellens der Ausgangsmaterialien eingesetzte flüssige oder pastöse oder feste Zusammensetzung, die mindestens ein Seidenprotein enthält, kann jede flüssige oder pastöse oder feste (bevorzugt flüssige oder pastöse) Zusammensetzung sein.

Die zur Beschichtung verwendete flüssige oder pastöse oder feste (bevorzugt flüssige oder pastöse) Zusammensetzung, die mindestens ein Seidenprotein enthält, kann ein Lösungsmittel oder Lösungsmittelgemisch enthalten. Bevorzugt ist das mindestens eine Seidenprotein in einem Lösungsmittel oder Lösungsmittelgemisch löslich oder bildet das mindestens eine Seidenprotein in einem Lösungsmittel ein Kolloid oder Hydrogel.

Ein Lösungsmittel kann ein kosmetisch verträgliches Lösungsmittel sein. Ein kosmetisch verträgliches Lösungsmittel kann beispielsweise ausgewählt sein aus der Gruppe bestehend aus Wasser, einem kosmetisch verträglichen Puffer, einem hydroalkoholischen Gemisch enthaltend Wasser und bis zu 5 Vol.-% Ethanol, Dimethylsulfoxid (DMSO), und Kombinationen aus zwei oder mehr daraus. Ein kosmetisch verträgliches Lösungsmittel kann optional ganz oder Teilweise auf dem implantierbaren Haar verbleiben. Alternativ kann es etwa durch Trocknung verdunstet werden.

Ein Lösungsmittel kann auch ein organisches Lösungsmittel sein. Dieses weist bevorzugt zumindest einen Grad an Polarität auf, der Löslichkeit oder Kolloidbildung des mindestens einen Seidenproteins ermöglicht. Beispielsweise kann ein organisches Lösungsmittel ausgewählt sein aus einem oder mehreren Alkoholen (z.B. Ethanol, Methanol, Propanol und/oder Butanol), Aceton, Acetonitril. Zumindest sofern das organische Lösungsmittel in der eingesetzten Konzentration nicht kosmetisch verträglich ist, ist das organische Lösungsmittel bevorzugt evaporierbar, kann also durch Verdunstung ganz oder teilweise entfernt werden.

Die zur Beschichtung verwendete flüssige oder pastöse oder feste (bevorzugt flüssige oder pastöse) Zusammensetzung kann neben einem Lösungsmittel optional auch ein oder mehrere Konservierungsmittel, ein oder mehrere antibakterielle (etwa bakterizide und/oder bakteriostatische) Mittel, ein oder mehrere antifungizide Mittel, ein oder mehrere antivirale Mittel, ein oder mehrere Detergenzien, ein oder mehrere Chelatoren, ein oder mehrere blutungsstillende Mittel, ein oder mehrere Vitamine, ein oder mehrere das Haarwachstum oder die Durchblutung fördernde bioaktive Stoffe, ein oder mehrere Lokalanästhetika, ein oder mehrere Verdickungsmittel, ein oder mehrere Hormone, ein oder mehrere Säureregulatoren oder eine Kombination aus zwei der mehr davon enthalten.

Die Begriffe "flüssig" und "pastös" sind wie im allgemeinen Sprachgebrauch verwendet zu verstehen. Bevorzugt weist eine flüssige Zusammensetzung bei Zimmertemperatur (20°C) unter Normaldruck (1013 mbar) eine Viskosität von weniger als 5000 mPa·s (z.B. bestimmt mit einem Kapillarviskosimeter) auf. Bevorzugt weist eine pastöse Zusammensetzung bei Zimmertemperatur (20°C) unter Normaldruck (1013 mbar) eine Viskosität von mindestens 5000 mPa·s (z.B. bestimmt mit einem Kapillarviskosimeter) auf.

Der Begriff "fest" ist im Weitesten Sinne zu verstehen als eine größere Festigkeit aufweisend als eine pastöse Zusammensetzung, daher bei Zimmertemperatur (20°C) unter Normaldruck (1013 mbar) nicht fließend. So kann etwa eine Seife flüssig oder pastös sein, oder aber als festes Seifenstück (auch: Handseife) vorliegen. Der Begriff "fest" kann auch wachsartig umfassen.

In einer Ausführungsform kann das Seidenprotein eine feste oder zähe pastöse (wachsartige) Struktur aufweisen. Dies kann gute technische Eigenschaften durch Pressen durch eine Injektionsnadel erzielen und Beschädigungen (etwa von Seidenprotein) bei der Injektion vermindern oder verhindern und somit die Schutzfunktion des Seidenproteins verbessern.

Eine solche feste oder zähe pastöse (wachsartige) Struktur kann optional erzielt werden, indem eine flüssige oder pastöse Zusammensetzung solange einer Temperatur- und/oder Lufttrocknung ausgesetzt wird, bis sie fest oder zäh-pastös (wachsartig) wird. Dies kann optional auch unter Zuhilfenahme von zusätzlichen chemischen Stoffen erfolgen.

Der Schritt (ii) des Inkontaktbringens kann auf jede dazu geeignete Weise erfolgen. Gemäß einer Ausführungsform der vorliegenden Erfindung umfasst der Schritt (ii) des Inkontaktbringens das Eintauchen des Haars oder eines Teils davon in die flüssige oder pastöse oder feste (bevorzugt flüssige oder pastöse) Zusammensetzung. Gemäß einer weiteren Ausführungsform der vorliegenden Erfindung umfasst der Schritt (ii) des Inkontaktbringens das Bestreichen des Haars oder eines Teils davon mit der flüssigen oder pastösen oder festen Zusammensetzung. Gemäß einer weiteren Ausführungsform der vorliegenden Erfindung umfasst der Schritt (ii) des Inkontaktbringens das Besprühen des Haars oder eines Teils davon mit der flüssigen oder pastösen oder festen Zusammensetzung. Es wird erkannt werden, dass optional auch Kombinationen unterschiedlicher Verfahrensschritte des Inkontaktbringens miteinander kombiniert werden können.

Gemäß einer Ausführungsform der vorliegenden Erfindung umfasst der Schritt (ii) des Inkontaktbringens das Zusammenführen eines Haars (oder Haarbündels) mit einer (weitgehend) festen Zusammensetzung, die ein oder mehrere Seidenproteine enthält. Gemäß einer Ausführungsform der vorliegenden Erfindung umfasst der Schritt (ii) des Inkontaktbringens das Einstecken eines Haars (oder Haarbündels) in eine vorgefertigte (weitgehend) feste Verankerungsstruktur. Gemäß einer Ausführungsform der vorliegenden Erfindung umfasst der Schritt (ii) des Inkontaktbringens das Umwickeln eines Haars (oder Haarbündels) mit einer oder mehreren Fasern und/oder eines oder mehrerer Bänder.

Es wird verstanden werden, dass auch mehrere Verfahrensschritte des Inkontaktbringens miteinander kombiniert werden können. Dies kann gleichzeitig und/oder zeitig hintereinander erfolgen. So kann beispielhaft eine Umwicklung oder ein Einstecken in eine (weitgehend) feste Verankerungsstruktur auch in einer flüssigen oder pastösen Zusammensetzung erfolgen.

Der Schritt (ii) des Inkontaktbringens kann bei jeder beliebigen Temperatur durchgeführt werden, bei der zumindest die Primärstruktur des eingesetzten mindestens einen Seidenproteins erhalten bleibt und nicht denaturiert wird, daher sich keine Aminosäurereste des mindestens einen Seidenproteins pyrolytisch zersetzen. Gemäß einer Ausführungsform der vorliegenden Erfindung wird der Schritt (ii) bei Temperaturen im Bereich von -40 bis 250°C, -20 bis 200°C, -10 bis 180°C, -5 bis 150°C, 0 bis 150°C, von 2 bis 120°C, von 3 bis 100°C, von 4 bis 80°C, von 4 bis 60°C, von 4 bis 50°C, von 4 bis 40°C, von 5 bis 30°C, von 10 bis 25°C oder von 15 bis 25°C durchgeführt. Gemäß einer Ausführungsform der vorliegenden Erfindung wird der Schritt (ii) bei Zimmertemperatur im Bereich von 15 bis 25°C oder von 18 bis 22°C, insbesondere (etwa) 20°C, durchgeführt.

Sofern eine andere Art des Inkontaktbringens zumindest eines implantierbaren Teils des Haars mit der flüssigen oder pastösen oder festen Zusammensetzung gemäß Schritt (ii), etwa mittels Bedampfung des Haars oder eines Teils davon (bei erhöhter Temperatur und/oder im Vakuum) gelingt, ist auch eine solche Vorgehensweise von der vorliegenden Erfindung umfasst. Auch kann die Beschichtung mit Seidenprotein seinerseits optional bedampft werden.

Der Schritt (ii) des Inkontaktbringens kann bei jedem beliebigen Druck durchgeführt werden. Bevorzugt wird der Schritt bei Umgebungsdruck durchgeführt, etwa im Bereich von 960 bis 1100 mbar, von 980 bis 1050 mbar oder von 990 bis 1040 mbar.

Der Schritt (ii) des Inkontaktbringens zumindest eines implantierbaren Teils des Haars mit der flüssigen oder pastösen oder festen Zusammensetzung kann das Inkontaktbringen eines terminalen Endes des Haars mit der flüssigen oder pastösen oder festen Zusammensetzung sein. Dann kann etwa ein terminales Ende eines einzelnen Haars in eine solche Zusammensetzung eingetaucht werden oder es kann ein terminales Ende eines zusammengefassten Büschel von mehreren Haaren in die Zusammensetzung eingetaucht werden. Auch kann etwa ein terminales Ende eines einzelnen Haars mit einer Zusammensetzung bestrichen werden oder es kann ein terminales Ende eines zusammengefassten Büschel von mehreren Haaren mit der Zusammensetzung bestrichen werden. Auch kann etwa ein terminales Ende eines einzelnen Haars mit einer Zusammensetzung besprüht werden oder es kann ein terminales Ende eines zusammengefassten Büschel von mehreren Haaren mit der Zusammensetzung besprüht werden.

Der Schritt (ii) des Inkontaktbringens zumindest eines implantierbaren Teils des Haars mit der flüssigen oder pastösen oder festen Zusammensetzung kann auch das Inkontaktbringen beider terminalen Enden des Haars mit der flüssigen oder pastösen oder festen Zusammensetzung sein. Dann kann das Haar etwa über einen Stab gezogen werden, so dass beide Enden auf einer Seite platziert sind, etwa herunterhängen, und beide Enden in die Zusammensetzung eintauchen oder von dieser bestrichen oder besprüht werden. Dies ist analog auch mit einem Bündel an mehreren Haaren möglich.

Gemäß einer bevorzugten Ausführungsform wird durch das Verfahren der vorliegenden Erfindung eine 3D-Oberflächenstruktur im Nano- oder Mikrometerbereich erhalten, die von einer weitgehend zylindrischen Form abweicht.

Das Abweichen von einer weitgehend zylindrischen Form ist regelmäßig technisch besonders vorteilhaft, da die Anhaftung eines implantierten Haars in die Kopfhaut besser ist. Das implantierte Haar kann fester und dauerhafter verankert werden.

Optional kann der Schritt (ii) des Inkontaktbringens zumindest eines implantierbaren Teils des Haars mit der flüssigen oder pastösen oder festen Zusammensetzung auch mittels 3D-Formgebung (z.B. 3D-Druck) erfolgen.

Gemäß einer bevorzugten Ausführungsform umfasst der Schritt (ii) des Inkontaktbringens das Anhaften der Zusammensetzung auf das Haar oder einen Teil davon mittels eines 3D-Formgebung-Verfahrens, wobei eine 3D-Oberflächenstruktur im Nano- oder Mikrometerbereich erhalten wird, die von der weitgehend zylindrischen Form des Haars (und bevorzugt auch einer Tropfenform) abweicht.

Gemäß einer bevorzugten Ausführungsform umfasst der Schritt (ii) des Inkontaktbringens das Anhaften der Zusammensetzung auf das Haar oder einen Teil davon mittels des teilweise Aufschmelzens der Zusammensetzung und/oder des mindestens einen Seidenproteins, insbesondere mittels eines Femtosekunden-Lasers, insbesondere im ultravioletten (UV) Spektralbereich.

Gemäß einer bevorzugten Ausführungsform umfasst das Verfahren den weiteren Schritt des teilweisen Entfernens von Seidenprotein, das auf das Haar aufgebracht worden ist.

Gemäß einer bevorzugten Ausführungsform erfolgt dies mittels Plasma-Ablation. Gemäß einer bevorzugten Ausführungsform erfolgt dies mittels eines Femtosekunden-Lasers, insbesondere im ultravioletten (UV) Spektralbereich.

Diese Verfahren sind oben weiter ausgeführt.

Gemäß einer bevorzugten Ausführungsform umfasst das Verfahren die folgenden Schritte:
(i) Bereitstellen von:
   (A) Haar, das sich nicht am menschlichen oder tierischen Körper befindet, und
   (B) einer weitgehend festen oder pastösen Zusammensetzung, die mindestens ein Seidenprotein enthält, die optional eine oder mehrere Fasern oder eine zumindest teilweise getrocknete Zusammensetzung gemäß der vorliegenden Erfindung darstellen kann;
(ii) Inkontaktbringen oder in Kontakt halten zumindest eines implantierbaren Teils des Haars mit der Zusammensetzung, wobei Schritte (i) und (ii) optional auch zeitgleich stattfinden können,
(iii) Anhaften der Zusammensetzung auf das Haar oder einen Teil davon mittels teilweisen Aufschmelzens der Zusammensetzung, die mindestens ein Seidenprotein enthält, bevorzugt durch einen Lichtpuls, insbesondere eines Femtosekunden-Lasers, insbesondere im ultravioletten (UV) Spektralbereich.

Der Schritt (ii) des Inkontaktbringens zumindest eines implantierbaren Teils des Haars mit der flüssigen oder pastösen oder festen Zusammensetzung kann auch das Inkontaktbringen eines Knicks oder knickbaren Teils des Haars mit der flüssigen oder pastösen oder festen Zusammensetzung sein. Dann kann das Haar etwa über einen Stab gezogen werden, so dass beide Enden auf einer Seite platziert sind, etwa herunterhängen, und die an der Biegung des Stabs befindliche Spitze in die Zusammensetzung eintaucht oder von dieser bestrichen oder besprüht wird. Dies ist analog auch mit einem Bündel an mehreren Haaren möglich.

Alternativ kann das Haar in Schritt (ii) auch vollständig in die flüssige oder pastöse oder feste Zusammensetzung, die mindestens ein Seidenprotein enthält, eintauchen oder vollständig damit bestrichen oder besprüht werden. Dies ist auch mit mehreren Haaren gleichzeitig möglich.

Optional kann das aus Schritt (ii) erhaltene Haar zur Implantation verwendet werden. In diesem Fall kann etwa die Beschichtung des Haars am Ort des Implantierens des Haars erfolgen oder in dessen räumlicher Nähe erfolgen.

Optional kann das aus Schritt (ii) erhaltene Haar auch einem zusätzlichen Schritt (iii) des Trocknens unterzogen werden. Dies kann die Lagerfähigkeit des implantierbaren Haars verlängern. Eine Trocknung kann unter beliebigen Bedingungen erfolgen. Bevorzugt werden die Bedingungen dabei so gewählt, dass das weder das Haar noch das mindestens eine Seidenprotein pyrolytisch zersetzt werden. Die Trocknung kann bei Raumtemperatur (15 bis 25°C, bevorzugt etwa 20°C) oder bei erhöhter Temperatur (etwa im Bereich von über 20°C bis 150°C oder von 30°C bis 100°C oder von 40°C bis 70°C) stattfinden. Die Trocknung kann bei Normaldruck oder bei vermindertem Druck (etwa im Vakuum) stattfinden. Die Trocknung kann auch eine Sprühtrocknung sein, wobei das beschichtete Haar aus Schritt (ii) tiefgefroren oder schockgefroren wird (bevorzugt bei Temperarturen <-10°C, <-20°C, <-60°C oder etwa -196°C) und dann im Vakuum das Lösungsmittel evaporiert wird. Die Trocknung kann optional auch in einem Luftstrom stattfinden. Die Trocknung kann optional auch mit einem Haartrockner beschleunigt werden.

Das erhaltene implantierbare Haar kann unter beliebigen geeigneten Bedingungen gelagert werden, etwa bei Umgebungstemperatur oder im Kühlschrank. Es kann optional verpackt werden. Es kann beispielsweise steril verpackt werden. Es kann beispielsweise im Vakuum oder unter Schutzgasatmosphäre verpackt werden. Es kann transparent oder lichtgeschützt verpackt werden. Es kann beispielsweise in einer flüssigen oder pastösen Zusammensetzung, die mindestens ein Seidenprotein enthält, verpackt und/oder gelagert werden.

Es wird verstanden werden, dass ein Seidenprotein, Haar und/oder das beschichtete Haar gemäß der vorliegenden Erfindung optional bei jeder dazu geeigneten Temperatur gelagert werden kann, etwa bei Temperaturen im Bereich von -200 bis 250°C, -100 bis 250°C, -80 bis 200°C, -50 bis 180°C, -10 bis 150°C, 0 bis 150°C, von 2 bis 120°C, von 3 bis 100°C, von 4 bis 80°C, von 4 bis 60°C, von 4 bis 50°C, von 4 bis 40°C, von 5 bis 30°C, von 10 bis 25°C oder von 15 bis 25°C. Bevorzugt wird es, wenn nicht bei Umgebungstemperatur gelagert, vor der Implantation auf eine Temperatur nahe der Umgebungstemperatur gebracht, etwa von 5 bis 30°C, von 10 bis 25°C oder von 15 bis 25°C. Wie in vielen Materialien durchlaufen Spinnenseidenfasern einen Glasübergang. Die Glasübergangstemperatur kann von der Luftfeuchtigkeit abhängen. Wasser kann hierbei als Weichmacher fungieren.

Eine Packung kann optional eine Verwendungsanleitung enthalten, die unter anderem lehrt, wie das implantierbare Haar in die Haut implantiert werden kann.

Daher wird hier auch Folgendes beschrieben: ein Kit (of parts) umfassend:
(A) implantierbares Haar gemäß der vorliegenden Erfindung; und
(B) eine Verwendungsanleitung zur Implantation;
sowie ein Kit umfassend:
(A) Haar;
(B) mindestens ein Behältnis enthaltend eine flüssige oder pastöse oder feste (bevorzugt flüssige oder pastöse) Zusammensetzung, die mindestens ein Seidenprotein enthält, die geeignet zur Beschichtung des Haars oder eines implantierbaren Teils davon ist;
(C) optional eine Verwendungsanleitung zum Beschichten des Haars mit der Zusammensetzung; und
(D) optional eine Verwendungsanleitung zur Implantation; und
ein Kit umfassend:
(A) Haar;
(B) mindestens ein Behältnis enthaltend ein Konzentrat oder Pulver zur Herstellung einer flüssigen oder pastösen oder festen (bevorzugt flüssige oder pastöse) Zusammensetzung, die mindestens ein Seidenprotein enthält, die geeignet zur Beschichtung des Haars oder eines implantierbaren Teils davon ist;
(C) optional eine Verwendungsanleitung zum Beschichten des Haars mit der Zusammensetzung;
(D) optional eine Verwendungsanleitung zur Implantation; und
(E) optional mindestens ein Lösungsmittel zur Herstellung der flüssigen oder pastösen Zusammensetzung aus dem Konzentrat oder Pulver.

Wie beschrieben kann das implantierbare Haar zum Implantieren in menschliche oder tierische Haut verwendet werden.

Daher betrifft ein weiterer Aspekt der vorliegenden Erfindung die kosmetische Verwendung des implantierbaren Haars zum Implantieren in menschliche oder tierische Haut, insbesondere menschliche Kopfhaut.

Somit umfasst ein weiterer Aspekt der vorliegenden Erfindung ein kosmetisches Verfahren zum Implantieren des implantierbaren Haars gemäß der vorliegenden Erfindung in menschliche oder tierische Haut, insbesondere menschliche Kopfhaut, umfassend die folgenden Schritte:
(i) Bereitstellen von Haar gemäß der vorliegenden Erfindung,
(ii) Einbringen des Haars in die menschliche oder tierische Haut, insbesondere menschliche Kopfhaut, bevorzugt mittels einer oder mehrerer Nadeln.

Es wird verstanden werden, dass alle im Zusammenhang mit dem implantierbaren Haar und dessen Herstellungsverfahren gemäß der vorliegenden Erfindung gemachten Definitionen und Ausführungsformen in entsprechender Weise auch für die Verwendung oder ein Verfahren zur Verwendung eines solchen implantierbaren Haars gelten.

Gemäß einer Ausführungsform erfolgt die Implantation in die Kopfhaut. Gemäß einer anderen Ausführungsform erfolgt die Implantation in eine oder mehrere andere Bereiche des Gesichts oder Körpers. So kann eine Implantation etwa im Bereich einer oder beider Augenbrauen, im Bereich einer oder beider Wimpern, im Bereich des Schnurrbarts, im Bereich des Kinnbarts, im Bereich des Backenbarts, im Bereich einer oder beider Koteletten, im Bereich der Achseln, im Bereich der Schamregion, im Bereich eines oder beider Arme, im Bereich der Brust, im Bereich eines oder beider Beine erfolgen. Je nach Einsatzgebiet kann entsprechendes Haar ausgewählt werden.

Die Implantation kann zu jedem beliebigen Zweck erfolgen. Typischerweise soll die Behaarung in einem oder mehreren Arealen der menschlichen oder Tierischen Haut gesteigert werden.

Gemäß einer Ausführungsform erfolgt die Implantation bevorzugt weitgehend symmetrisch auf beiden Körperhälften. Bei einem Unfall und/oder einer Narbenbildung kann es jedoch auch angezeigt sein, eine Körperseite stärker oder sogar ausschließlich einer Implantation zu unterziehen. Die Implantation kann zur Herstellung oder Wiederherstellung von Kopfbehaarung erfolgen. Auch zur Hervorhebung männlicher Körpermerkmale kann eine Implantation von Haaren auch als Körperbehaarung von Interesse sein, so beispielsweise bei einer Geschlechtsumwandlung. Auch können längere Wimpern implantiert werden. Auch eine bestimmte Form eines Bartes kann durch Implantation von implantierbarem Haar gemäß der vorliegenden Erfindung erreicht werden. Die Implantation der erfindungsgemäßen implantierbaren Haare kann auch verwendet werden für die Rekonstruktion von Augenbrauen, Wimperntransplantation nach Alopecia mechanica, Alopecia areata, Bartersatz etwa nach einer Korrektur einer Hasenscharte und/oder (optional narbige traumatische) Alopezie.

Optional kann zusätzlich zu einer erfindungsgemäßen Implantation von Haaren auch noch eine Behaarung simuliert werden und/oder das teilweise oder vollständige Fehlen von Haaren auf andere Weise kaschiert werden. So können etwa Augenbrauen auch aufgeschminkt/aufgemalt oder mit Schminke nachgezeichnet werden. Auch eine Tätowierung der optischen Erscheinung von kurzen Haaren und/oder die Färbung der Kopfhaut kommt in Betracht.

Das implantierte implantierbare Haar gemäß der vorliegenden Erfindung kann optional dauerhaft in der Haut des Empfängers anwachsen. Es kann optional auch im Rahmen der Hauterneuerung zur Epidermis wandern und schließlich ausfallen. Die Dauer des Verbleibs in der Haut kann über die Verankerung wie oben beschrieben definiert werden. So kann der natürliche Lebenszyklus des Haars simuliert werden. Die Halbwertszeit kann gemeinsam mit dem Empfänger gewählt werden.

So kann die Halbwertszeit des implantierten Haars gewählt werden. Der Haarausfall kann durch haptischen und mechanischen Einfluss wie etwa Kämmen, Waschen der entsprechenden Kopf- oder Körperpartie, Wind, Haartrocknung, reiben durch Kleidung usw. gesteigert und beschleunigt werden.

Die Implantation kann im Prinzip über jedes bekannte Verfahren durchgeführt werden. Dem Fachmann sind zahlreiche Verfahren dem Grunde nach bekannt. Die nachfolgenden Figuren, Ansprüche und Beispiele beschreiben weitere Ausführungsformen der Erfindung, welche die Erfindung weiter veranschaulichen.

### Beschreibung der Figuren

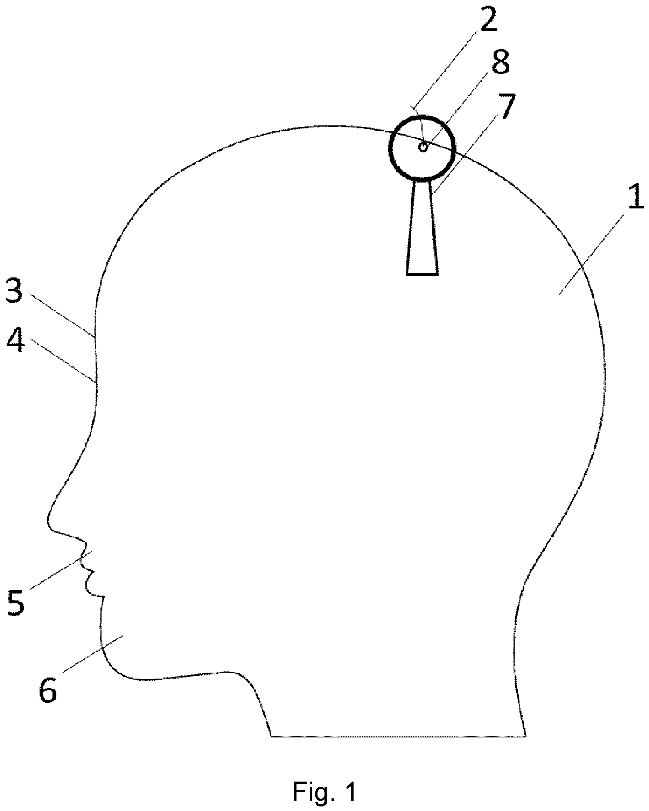

**Figur 1** zeigt schematisch die Haarimplantation eines oder mehrerer Haare (2) auf in die Kopfhaut des Kopfes (1) eines Menschen. Es wird zudem gezeigt, dass die Haarverpflanzung auch im Bereich der Augenbraue (3), der Wimpern (4), des Schnurrbarts (5) oder des übrigen Barts (6) (z.B., Kinn- oder Backenbart oder Koteletten) erfolgen kann. Ebenso kann die Haarimplantation auch an sonstigen Stellen des Körpers durchgeführt werden. Zur besseren Veranschaulichung wird die Haartransplantation durch eine Lupe (7) betrachtet dargestellt, wobei die Beschichtung des implantierbaren Teils (hier exemplarisch des implantierbaren terminalen Endes) eines Haars (2) in Figur 1 exemplarisch als Nachbildung einer Haarwurzel oder eines Haarfollikels oder einer Haarzwiebel (8) umfassend Seidenprotein (z.B. Spinnenseide und/oder Raupenseide) dargestellt wird.
**Figuren 2 bis 27** zeigen Beispiele für Strukturen, die der Verankerung des erfindungsgemäßen implantierbaren Haars (2) dienen können. So kann etwa eine als Nachbildung einer Haarwurzel oder eines Haarfollikels oder Haarzwiebel (8) gebildete Struktur umfassend Seidenprotein (z.B. Spinnenseide und/oder Raupenseide) eingesetzt werden, die optional eine zusätzliche Ankerstruktur (9) umfasst. Auch kann eine Struktur, die der Verankerung des erfindungsgemäßen implantierbaren Haars (2) dienen kann, einen oder mehrere Einschnitte (10) aufweisen und dadurch eine verbesserte Verankerung erzielen. Wie in den Figuren erkennbar ist, kann eine Verankerung optional eine zusätzliche Ankerstruktur (9) aus einer Zusammensetzung umfassend Seidenprotein (z.B. Spinnenseide und/oder Raupenseide) oder damit beschichtet enthalten, welche die Funktion von Widerhaken, einer Spiralform wie bei einer Schraube oder Ausstülpungen (9) haben, um die Befestigung des Haarimplantats zu ermöglichen oder zu verbessern. Auch kann die Beschichtung, die optionale Verankerung und/oder die optionalen Strukturen an der Verankerung verschiedene Festigkeiten haben. Zudem kann die Verankerung (9) ringförmig sein, wodurch das Einwachsen des umliegenden Gewebes verbessert werden kann. Auch kann die Verankerung (9) optional ein oder mehrere Anhängsel haben mit einer Zusammensetzung umfassend Seidenprotein (z.B. Spinnenseide und/oder Raupenseide) enthaltend, die mit einer Art Brücke oder jede andere Form von Verbindung mit der Verankerung verbunden sind. Auch kann die Verankerung eine Struktur aufweisen, bspw. wabenförmig, die einen höheren Härtegrad haben kann als die umliegende Verankerung. Das implantierbare Haar (2) kann optional vollständig oder teilweise, bevorzugt an dessen implantierbarem Teil, eine Ummantelung (11) enthalten, die aus einer Zusammensetzung besteht, die Seidenprotein (z.B. Spinnenseide und/oder Raupenseide) umfasst. Auch kann ein implantierbares Haar (2) eine natürliche/echte Haarwurzel (12) und/oder Haarfollikel und/oder Haarzwiebel umfassen, die mit einer Beschichtung (13) mit einer Zusammensetzung umfassend Seidenprotein (z.B. Spinnenseide und/oder Raupenseide) umgeben sein kann.

### Beispiel

Die Spinnenseide kann auf verschiedene Arten gewonnen werden. Beispielhaft wird ein Seidenprotein wie in WO 2007/025719 und WO 2006/008163 beschrieben verwendet, daher ein Fibroin-3 und/oder -4 der Europäischen Gartenkreuzspinne *(Araneus diadematus).* Ein solches wird auch als *"Araneus diadematus* Fibroin 3" (ADF-3) bzw. *Araneus diadematus* Fibroin 4" (ADF-4) oder auch als ("Major Ampullate Spidroin II" oder "Spidroin 2") bezeichnet. Das ADF-3 und/oder ADF-4 Spinnenseidenprotein, welches hier beispielhaft beschrieben wird, kann kommerziell erhalten werden. Es wurde wie in WO 2006 008163 beschrieben hergestellt. Daher wurde in einem bakteriellen Fermentationsprozess durch *Escherichia coli* Bakterien (auch E. *coli* oder Kolibakterium genannt) hergestellt. In die E. coli-Bakterien wurde durch Gentransfer Spinnenseideprotein-kodierende DNA der Europäischen Gartenkreuzspinne *(Araneus diadematus)* eingesetzt. Dabei wurden genetisch veränderte E. coli-Bakterien geschaffen, die Spinnenseidenproteine exprimieren, indem sie beispielsweise Rüben oder Zuckerrohr fermentierten.

Das implantierbare Haar für die Implantation stammt vorwiegend aus Indien. Unter Hindu-Pilgern ist es üblich, nach einem Gelübde oder bei einem neuen Lebensabschnitt ihre Haare zu opfern. Dies geschieht vorwiegend in indischen Tempeln, weshalb das Haar auch als Tempelhaar bezeichnet wird. Um die Haare beispielsweise für den europäischen Markt aufzubereiten, werden sie nach ihren Eigenschaften, beispielsweise der Länge nach, sortiert, gebündelt, gebleicht und gefärbt.

Für die Implantation des implantierbaren Haars kann aber auch Eigenhaar abgeschnitten werden. Um eine gute Qualität zu gewährleisten sollte es möglichst ungebleicht und ungefärbt sein. Zur Beschichtung des implantierbaren Haars wird es sortiert und in schmalen Streifen gebündelt arretiert. Am terminalen Haarende werden die Haare mit einer sehr scharfen Klinge abgeschnitten, damit sie alle an der gleichen Stelle enden. Dadurch wird sichergestellt, dass jedes einzelne Haar mit Seide beschichtet wird. Danach erfolgt die Beschichtung mit Seide durch Eintauchen des implantierbaren Teils des Haars in die flüssige oder pastöse Zusammensetzung oder Reiben an der festen Zusammensetzung. Zur besseren Verteilung der Seide wird das Haarbündel während des Eintauchens in die flüssige oder pastöse Zusammensetzung rotiert.

Danach werden die Haare der Trocknung ausgesetzt. Zur Beschleunigung der Trocknung werden sie einer leichten Luftventilation ausgesetzt. Zur Bildung weiterer Seidenbeschichtungen werden die Haare nach der Trocknung der Seidenschicht nach dem gleichen Prinzip erneut in die flüssige oder pastöse Zusammensetzung eingetaucht.

Diese Prozessschritte werden so oft wiederholt, bis die gewünschte Dicke der Schutzschicht um das implantierbare Haar und der Verankerung erreicht wurde. Um zusätzlich beispielsweise eine pilzförmige Verankerung herzustellen, wird das zu implantierende Haarende ab einer gewissen Prozesswiederholung nur bis zu der Stelle in die flüssige oder pastöse Zusammensetzung eingetaucht, an der die pilzhutförmige Verbreiterung entstehen soll.

Vor der Implantation wird die Kopfhaut zunächst lokal betäubt. Dies geschieht mit einem Kolben, in welchen das Lokalanästhetikum gefüllt wird, wie etwa mittels des Injektionsgeräts "Comfort-in" (Mika Medical Co. 93, Noksansaneopjung-ro, Gangseo-gu,Busan, Republik Korea). Dabei verfügt der Kolben am unteren Ende über eine Düse mit einer Öffnung von ca. 0,15 mm. Sobald der Auslöser gedrückt wird, hat der Kolben etwa 0,3 Sekunden Kontakt mit der Haut und bringt in dieser kurzen Zeitspanne das Betäubungsmittel mit Hochdruck unter die Haut, wodurch sie unverletzt bleibt. Dabei werden alle Hautschichten betäubt.

Um das implantierbare Haar in die Haut, insbesondere der Kopfhaut, zu implantieren, wird ein spezielles im Stand der Technik zu diesem Zweck bekanntes Gerät verwendet, das einem Druckbleistift ähnelt. Hierzu können etwa klassische CII Hohlnadeln (Punches) von Cole Instruments Inc. (1070 Powers Place, Alpharetta GA 30009 USA) mit entsprechenden handelsüblichen Zusatzgeräten verwendet werden. Eine derartige Vorrichtung umfasst eine äußere Hohlnadel, mit welcher die Haut durchstochen wird und eine innere Hohlnadel, in welche das Implantationshaar gebracht und durch eine Klemmmechanik arretiert wird. An der arretierten Stelle ist eine Vorschubmechanik (ebenfalls ähnlich einem Druckbleistift) mit deren Hilfe das Implantationshaar am terminalen Ende ca. 1 mm aus der Spitze der inneren Hohlnadel geschoben wird. Der elektrische Auslösemechanismus, um die innere Hohlnadel vorzuschieben und die Arretierung zu lösen, wird dabei durch Betätigen eines Pedals mit dem Fuß gesteuert.

Der Innendurchmesser der inneren Hohlnadel kann dabei in etwa 0,05 bis 0,6 mm breiter sein als die Verankerung des Haarimplantats. Der Innendurchmesser der äußeren Hohlnadel ist nur soweit breiter, wie es erforderlich ist, damit die innere Hohlnadel durchgleiten kann. Der Außendurchmesser der äußeren Hohlnadel ist in etwa 0,2 bis 0,9 mm breit.

Das Implantationsgerät wird in Haarwachsrichtung an die Haut angelehnt mit einem Winkel, der dem natürlichen Austrittswinkel der Haare entspricht, in etwa 45 Grad. Die äußere Hohlnadel wird in die Haut gebracht. Dann wird mit dem Pedal der Auslöser gedrückt, wodurch die innere Hohlnadel automatisch durch die äußere Hohlnadel nach vorne geschoben wird. Sobald die innere Hohlnadel die Spitze der äußeren Hohlnadel erreicht, wird das Haar mit der Vorschubmechanik nach vorne geschoben. Sobald das Haar wie beschrieben vorgeschoben ist, wird die Klemmmechanik automatisch gelöst und das Haar freigegeben. Dann wird das Implantationsgerät wieder aus der Haut gebracht und für die Implantation des nächsten Haares vorbereitet.

Ein alternatives Verfahren ist die Implantation des Haars mit einem Gerät, wie es bereits bei der Transplantation von Eigenhaaren verwendet wird. Zu diesem Zweck kann beispielsweise der "OKT Implanter" des Herstellers Cole Instruments Inc. (1070 Powers Place, Alpharetta GA 30009 USA) eingesetzt werden, dessen Nadel einen Durchmesser von 0,6 bis 1,14 mm haben kann. Bei der Transplantation von Eigenhaar wird das entnommene Haar, samt Haarwurzel, in eine Nadel gelegt, welche an einer Seite einen Einschnitt bzw. eine durchgehende Öffnung hat. Dann wird das Haar in die Kopfhaut gedrückt und dabei implantiert. Auch bei der Implantation von erfindungsgemäßem implantierbarem Haar mit einer Beschichtung, die mindestens ein Seidenprotein enthält, und optional eine Verankerungsstruktur aufweist, kann das Haar samt Verankerung in die Nadel des OKT Implanters gelegt, in die Haut gedrückt und dabei implantiert werden. Das Haar kann dabei an der Öffnung der Nadel heraushängen, wodurch es möglich ist auch langes Haar zu implantieren.

In Abhängigkeit von der Anzahl der zu implantierenden implantierbaren Haare, kann eine Behandlung einige Stunden bis einige Tage dauern.

## Patentansprüche

1. Implantierbares Haar, **dadurch gekennzeichnet, dass** es zumindest an einem implantierbaren Teil mit einer direkt mit dem Haar in Kontakt kommenden Zusammensetzung beschichtet ist, die mindestens ein Seidenprotein enthält.

2. Implantierbares Haar gemäß Anspruch 1, wobei das mindestens eine Seidenprotein mindestens 90% Sequenzhomologie zu einem Spinnenseidenprotein oder einem Insektenseidenprotein aufweist, bevorzugt wobei das mindestens eine Seidenprotein mindestens 90% Sequenzhomologie zu einem Seidenprotein aus einer der Gattungen *Araneus, Bombyx, Nephila, Antheraea, Arachnura, Caerostris, Argiope, Cyrtophora, Celaenia, Gasteracantha, Ordgarius, Neoscona, Zygiella, Parawixia, Neoscona, Dolophones, Aculeperia, Eriophora, Anepsion, Tegenaria, Heurodes, Cyclosa, Astracantha, Eriovixia, Nephilengys, Herennia, Acusilas, Neoscona, Poltys, Arkys, Poecilopachys Hyalophora, Samia, Attacus, Circula, Gonometa* und/oder *Anaphe* aufweist, stärker bevorzugt wobei das mindestens eine Seidenprotein mindestens 90% Sequenzhomologie zu einem Seidenprotein ausgewählt einer Spezies aus der Gruppe bestehend aus *Bombyx mori, Bombyx mandarina, Antheraea pernyi, Antheraea yamamai, Antheraea mylitta, Antheraea roylei, Antheraea proyeli, Antheraea paphia, Antheraea frithi, Antheraea assama, Araneus didenatus, Nephila clavipes, Araneus bicentenarius, Arachnura higginsi, Araneus circulissparsus, Araneus diadematus, Caerostris darwini, Argiope picta, Argiope trifasciata Nephila antipodiana, Cyrtophora beccarii, Celaenia excavata, Gasteracantha kuhlii, Argiope aurantia, Ordgarius furcatus, Ordgarius magnificus, Neoscona nautica, Neoscona rufofemorata, Zygiella calyptrata, Parawixia dehaani, Neoscona oxancensis, Gasteracantha cancriformis, Gasteracantha arcuata, Cyrtophora moluccensis, Cyrtophora parnasia, Dolophones conifera, Dolophones turrigera, Gasteracantha doriae, Gasteracantha mammosa, Cyrtophora exanthematica, Aculeperia ceropegia, Eriophora pustulosa, Anepsion depressium, Gasteracantha quadrispinosa, Eriophora transmarina, Araneus bicentenarius, Nephila maculata, Gasteracantha hasseltii, Tegenaria atrica, Heurodes turrita, Cyclosa insulana, Astracantha minax, Araneus mitificus, Eriovixia laglaisei, Cyclosa bifida, Nephilengys malabarensis, Argiope versicolor, Herennia ornatissima, Argiope aemula, Cyrtophora unicolor, Cyrtophora hirta, Argiope keyserlingi, Acusilas coccineus, Argiope argentata, Gasteracantha cancriformis, Neoscona domiciliorum, Argiope aetheria, Argiope Keyserlingi, Poltys illepidus, Arkys clavatus, Arkys lancearius, Poecilopachys australasia, Nephila clavipes, Nephila senegalensis, Nephila madagascariensis, Hyalophora cecropia, Samia Samia cynthia, Attacus atlas, Circula trifenestrata, Gonometa postica, Gonometa rufobrunnea, Anaphe panda* und/oder *Anaphe moloneyi* aufweist, insbesondere wobei das mindestens eine Seidenprotein mindestens 90% Sequenzhomologie zu einem Seidenprotein einer Spezies ausgewählt aus der Gruppe bestehend aus *Bombyx mori, Antheraea pernyi, Araneus diadematus, Nephila clavipes, Araneus bicentenarius* und *Caerostris darwini* aufweist.

3. Implantierbares Haar gemäß mindestens einem der Ansprüche 1 oder 2, wobei das mindestens eine Seidenprotein **dadurch gekennzeichnet ist, dass**:
(a) es ausgewählt ist aus der Gruppe bestehend aus Fibroin, Sericin, Spidroin 1 und Spidroin 2; und/oder
(b) es mindestens zwei Sequenzabschnitte umfasst, die jeweils eine der folgenden Sequenzen SEQ ID NO: 1 bis 6 aufweisen:
| | |
|---|---|
| GPGXX | (SEQ ID NO: 1), |
| GVPGX | (SEQ ID NO: 2), |
| GSGXX | (SEQ ID NO: 3), |
| GGYXX | (SEQ ID NO: 4), |
| PQQXX | (SEQ ID NO: 5), |
| GYGXX | (SEQ ID NO: 6), |
wobei jedes X unabhängig voneinander jede beliebige natürliche Aminosäure sein kann, vorzugsweise ausgewählt ist aus der Gruppe bestehend aus V, G, A, S, Y, P und Q.

4. Implantierbares Haar gemäß mindestens einem der Ansprüche 1 bis 3, wobei der mindestens eine implantierbare Teil ein implantierbares terminales Ende oder ein implantierbarer Knick in der Mitte des Haars oder ein knickbarer Teil in der Mitte des Haars ist.

5. Implantierbares Haar gemäß mindestens einem der Ansprüche 1 bis 4, wobei das implantierbare Haar **dadurch gekennzeichnet ist, dass** es:
(a) ein menschliches Haar ist;
(b) ein abgeschnittenes menschliches Haar ohne Haarfollikel und ohne Haarwurzel und ohne Haarzwiebel ist;
(c) ein synthetisches Haar oder ein tierisches Haar ist; und/oder
(d) über die gesamte Länge einen mittleren Durchmesser von 0,02 bis 0,15 mm aufweist.

6. Implantierbares Haar gemäß mindestens einem der Ansprüche 1 bis 5, wobei das implantierbare Haar durch mindestens eines der folgenden Merkmale gekennzeichnet ist:
(1) wobei das implantierbare Haar an dem implantierbaren Teil keine Verdickung aufweist, die einen maximalen Umfang von mehr als dem Zweifachen des mittleren Umfangs des Haars entspricht;
(2) wobei das implantierbare Haar an dem implantierbaren Teil eine künstliche Verdickung aufweist, die durch die Zusammensetzung gebildet wird, wobei die Verdickung einen maximalen Umfang um das Haar herum von mindestens dem Zweifachen des mittleren Umfangs des Haars entspricht;
(3) wobei das implantierbare Haar an dem implantierbaren Teil eine künstliche Verankerungsstruktur aufweist, die optional ausgewählt sein kann aus der Gruppe bestehend aus der Form eines Kegels, eines stumpfen Kegels, eines verdickten Zylinders, eines Pilzes, eines Tropfens, eines Ellipsoids, eines Quaders, einer Spiralform, einer Schraubenform, einer Ringform, einer Kugel, einer Wabenstruktur, einer teilweisen Umwicklung des Haars mit einer Faser oder einem Band und einer Kombination aus zwei oder mehr daraus und optional von der Haarstruktur und/oder der Verankerungsstruktur abstehende Fortsätze aufweisen kann;
(4) wobei das implantierbare Haar an dem implantierbaren Teil eingeschnitten ist;
(5) wobei das implantierbare Haar an dem implantierbaren Teil ein Haarfollikel oder eine Haarwurzel oder eine Haarzwiebel aufweist, die mit der Zusammensetzung beschichtet wird;
(6) wobei zwei oder mehr implantierbare Haare an ihren implantierbaren Teilen in einer Beschichtungs- oder Verankerungsstruktur zusammengefasst sind;
(7) wobei die Beschichtung Zonen mit unterschiedlicher Festigkeit und/oder biologischer Stabilität aufweist, insbesondere im Inneren nahe am Haar eine höhere Festigkeit und/oder biologische Stabilität aufweist als an der Außenseite,
(8) wobei die Beschichtung des implantierbaren Haars, umfassend das mindestens eine Seidenprotein, so gestaltet ist, dass nach einer Implantation das Seidenprotein oder ein Abbauprodukt auf das außerhalb des Körpers befindliche Haar und/oder die Kopfhaut abtransportiert wird und dort das Haar und/oder die Haut pflegt.

7. Implantierbares Haar gemäß mindestens einem der Ansprüche 1 bis 6, wobei das implantierbare Haar mittels 3D-Formgebung, insbesondere 3D-Druck, mit der Zusammensetzung beschichtet wurde,
insbesondere wobei dessen Herstellung das zumindest teilweise Aufschmelzen der Zusammensetzung und/oder des mindestens einen Seidenproteins umfasst, insbesondere mittels eines Femtosekunden-Lasers.

8. Implantierbares Haar gemäß einem der Ansprüche 1 bis 7, wobei dessen Herstellung das teilweise Entfernen von Seidenprotein, das auf das Haar aufgebracht worden ist, umfasst, insbesondere mittels Plasma-Ablation, insbesondere mittels eines Femtosekunden-Lasers.

9. Implantierbares Haar gemäß einem der Ansprüche 1 bis 8, wobei das implantierbare Haar zumindest teilweise mit einer festen oder pastösen Zusammensetzung beschichtet ist, die eine 3D-Oberflächenstruktur im Nano- oder Mikrometerbereich aufweist, die von einer weitgehend zylindrischen Form abweicht.

10. Verfahren zum Herstellen implantierbaren Haars gemäß mindestens einem der Ansprüche 1 bis 9, wobei das Verfahren die folgenden Schritte umfasst:
(i) Bereitstellen von:
(A) Haar, das sich nicht am menschlichen oder tierischen Körper befindet, und
(B) einer Zusammensetzung, die mindestens ein Seidenprotein enthält; und
(ii) Inkontaktbringen zumindest eines implantierbaren Teils des Haars mit der Zusammensetzung.

11. Verfahren gemäß Anspruch 10, wobei der Schritt (ii) des Inkontaktbringens umfasst:
(a) das Eintauchen des Haars oder eines Teils davon in die flüssige oder pastöse Zusammensetzung;
(b) das Bestreichen oder Besprühen des Haars oder eines Teils davon mit der flüssigen oder pastösen oder festen Zusammensetzung;
(c) das Anhaften der Zusammensetzung auf das Haar oder einen Teil davon mittels eines 3D-Formgebung-Verfahrens, wobei eine 3D-Oberflächenstruktur im Nano- oder Mikrometerbereich erhalten wird, die von einer weitgehend zylindrischen Form abweicht.
(d) das Anhaften der Zusammensetzung auf das Haar oder einen Teil davon mittels des teilweisen Aufschmelzens der Zusammensetzung und/oder des mindestens einen Seidenproteins, insbesondere mittels eines Femtosekunden-Lasers.
(e) das Verfahren den weiteren Schritt des teilweisen Entfernens von Seidenprotein, das auf das Haar aufgebracht worden ist, insbesondere mittels Plasma-Ablation, insbesondere mittels eines Femtosekunden-Lasers, wobei bevorzugt eine 3D-Oberflächenstruktur im Nano- oder Mikrometerbereich erhalten wird, die von einer weitgehend zylindrischen Form abweicht.

12. Verfahren gemäß einem der Ansprüche 10 oder 11, wobei das Verfahren die folgenden Schritte umfasst:
(i) Bereitstellen von:
(A) Haar, das sich nicht am menschlichen oder tierischen Körper befindet, und
(B) einer flüssigen oder pastösen oder festen Zusammensetzung, die mindestens ein Seidenprotein enthält;
(ii) Inkontaktbringen zumindest eines implantierbaren Teils des Haars mit der flüssigen oder pastösen oder festen Zusammensetzung; und
(iii) optional Trocknen des aus Schritt (ii) erhaltenen implantierbaren Haars.
und/oder wobei das Verfahren die folgenden Schritte umfasst:
(i) Bereitstellen von:
(A) Haar, das sich nicht am menschlichen oder tierischen Körper befindet, und
(B) einer weitgehend festen oder pastösen Zusammensetzung, die mindestens ein Seidenprotein enthält, die optional eine oder mehrere Fasern oder eine zumindest teilweise getrocknete Zusammensetzung gemäß Anspruch 11 darstellen kann;
(ii) Inkontaktbringen oder in Kontakt halten zumindest eines implantierbaren Teils des Haars mit der Zusammensetzung, wobei Schritte (i) und (ii) optional auch zeitgleich stattfinden können,
(iii) Anhaften der Zusammensetzung auf das Haar oder einen Teil davon mittels teilweisen Aufschmelzens der Zusammensetzung, die mindestens ein Seidenprotein enthält, bevorzugt durch einen Lichtpuls, insbesondere eines Femtosekunden-Lasers,
wobei bevorzugt eine 3D-Oberflächenstruktur im Nano- oder Mikrometerbereich erhalten wird, die von einer weitgehend zylindrischen Form abweicht.

13. Kosmetische Verwendung implantierbaren Haars gemäß mindestens einem der Ansprüche 1 bis 9 zum Implantieren in menschliche oder tierische Haut, insbesondere menschliche Kopfhaut.

14. Kosmetisches Verfahren zum Implantieren implantierbaren Haars gemäß mindestens einem der Ansprüche 1 bis 9 in menschliche oder tierische Haut, insbesondere menschliche Kopfhaut, umfassend die folgenden Schritte:
(i) Bereitstellen von Haar gemäß mindestens einem der Ansprüche 1 bis 9;
(ii) Einbringen des Haars in die menschliche oder tierische Haut, insbesondere menschliche Kopfhaut, mittels einer Nadel.

## Claims

1. Implantable hair, **characterized in that** it is coated at least in an implantable section with a composition that comes into direct contact with the hair and contains at least one silk protein.

2. Implantable hair according to Claim 1, wherein the at least one silk protein has at least 90% sequence homology to a spider silk protein or an insect silk protein, preferably wherein the at least one silk protein has at least 90% sequence homology to a silk protein from one of the genera *Araneus, Bombyx, Nephila, Antheraea, Arachnura, Caerostris, Argiope, Cyrtophora, Celaenia, Gasteracantha, Ordgarius, Neoscona, Zygiella, Parawixia, Neoscona, Dolophones, Aculeperia, Eriophora, Anepsion, Tegenaria, Heurodes, Cyclosa, Astracantha, Eriovixia, Nephilengys, Herennia, Acusilas, Neoscona, Poltys, Arkys, Poecilopachys Hyalophora, Samia, Attacus, Circula, Gonometa* and/or *Anaphe,* more preferably wherein the at least one silk protein has at least 90% sequence homology to a silk protein selected from a species from the group consisting of *Bombyx mori, Bombyx mandarina, Antheraea pernyi, Antheraea yamamai, Antheraea mylitta, Antheraea roylei, Antheraea proyeli, Antheraea paphia, Antheraea frithi, Antheraea assama, Araneus didenatus, Nephila clavipes, Araneus bicentenarius, Arachnura higginsi, Araneus circulissparsus, Araneus diadematus, Caerostris darwini, Argiope picta, Argiope trifasciata, Nephila antipodiana, Cyrtophora beccarii, Celaenia excavata, Gasteracantha kuhlii, Argiope aurantia, Ordgarius furcatus, Ordgarius magnificus, Neoscona nautica, Neoscona rufofemorata, Zygiella calyptrata, Parawixia dehaani, Neoscona oxancensis, Gasteracantha cancriformis, Gasteracantha arcuata, Cyrtophora moluccensis, Cyrtophora parnasia, Dolophones conifera, Dolophones turrigera, Gasteracantha doriae, Gasteracantha mammosa, Cyrtophora exanthematica, Aculeperia ceropegia, Eriophora pustulosa, Anepsion depressium, Gasteracantha quadrispinosa, Eriophora transmarina, Araneus bicentenarius, Nephila maculata, Gasteracantha hasseltii, Tegenaria atrica, Heurodes turrita, Cyclosa insulana, Astracantha minax, Araneus mitificus, Eriovixia laglaisei, Cyclosa bifida, Nephilengys malabarensis, Argiope versicolor, Herennia ornatissima, Argiope aemula, Cyrtophora unicolor, Cyrtophora hirta, Argiope keyserlingi, Acusilas coccineus, Argiope argentata, Gasteracantha cancriformis, Neoscona domiciliorum, Argiope aetheria, Argiope Keyserlingi, Poltys illepidus, Arkys clavatus, Arkys lancearius, Poecilopachys australasia, Nephila clavipes, Nephila senegalensis, Nephila madagascariensis, Hyalophora cecropia, Samia Samia cynthia, Attacus atlas, Circula trifenestrata, Gonometa postica, Gonometa rufobrunnea, Anaphe panda and*/*or Anaphe moloneyi,* especially wherein the at least one silk protein has at least 90% sequence homology to a silk protein from a species selected from the group consisting of *Bombyx mori, Antheraea pernyi, Araneus diadematus, Nephila clavipes, Araneus bicentenarius* and *Caerostris darwini.*

3. Implantable hair according to at least one of Claims 1 or 2, wherein the at least one silk protein is **characterized in that**:
(a) it is selected from the group consisting of fibroin, sericin, spidroin 1 and spidroin 2; and/or
(b) it comprises at least two sequence sections each having one of the following sequences SEQ ID NO: 1 to 6:
GPGXX (SEQ ID NO: 1),
GVPGX(SEQ ID NO: 2),
| | |
|---|---|
| GSGXX | (SEQ ID NO: 3), |
| GGYXX | (SEQ ID NO: 4), |
| PQQXX | (SEQ ID NO: 5), |
| GYGXX | (SEQ ID NO: 6), |
where X may be any natural amino acid, and is preferably selected from the group consisting of V, G, A, S, Y, P and Q.

4. Implantable hair according to at least one of Claims 1 to 3, wherein the at least one implantable section is an implantable terminal end or an implantable kink in the middle of the hair or a kinkable portion in the middle of the hair.

5. Implantable hair according to at least one of Claims 1 to 4, wherein the implantable hair is **characterized in that**:
(a) it is a human hair;
(b) it is a human hair that has been cut off without the hair follicle and without the hair root and without the hair bulb;
(c) it is a synthetic hair or an animal hair; and/or
(d) over the entire length it has an average diameter of 0.02 to 0.15 mm.

6. Implantable hair according to at least one of Claims 1 to 5, wherein the implantable hair is **characterized by** at least one of the following features:
(1) wherein the implantable hair, in the implantable section, has no thickening corresponding to a maximum circumference of more than twice the average circumference of the hair;
(2) wherein the implantable hair, in the implantable section, has an artificial thickening which is formed by the composition, wherein the thickening corresponds to a maximum circumference around the hair of at least twice the average circumference of the hair;
(3) wherein the implantable hair, in the implantable section, has an artificial anchoring structure that may optionally be selected from the group consisting of the form of a cone, a frustocone, a thickened cylinder, a mushroom, a droplet, an ellipsoid, a cuboid, a spiral form, a screw form, a ring form, a sphere, a honeycomb structure, a partial wrapping of the hair with a fibre or a tape, and a combination of two or more of these, and may optionally have appendages that project from the hair structure and/or the anchoring structure;
(4) wherein the implantable hair is notched in the implantable section;
(5) wherein the implantable hair, in the implantable section, has a hair follicle or a hair root or a hair bulb coated with the composition;
(6) wherein two or more implantable hairs, in their implantable sections, are encompassed in a coating or anchoring structure;
(7) wherein the coating has zones with different strength and/or biological stability, and especially has higher strength and/or biological stability in the interior close to the hair than on the outside,
(8) wherein the coating of the implantable hair, comprising the at least one silk protein, is designed such that, after implantation, the silk protein or a degradation product is transported onto the hair outside the body and/or the scalp, where it cares the hair and/or the skin.

7. Implantable hair according to at least one of Claims 1 to 6, wherein the implantable hair has been coated with the composition by means of 3D shaping, especially 3D printing, in particular wherein the production thereof comprises the at least partial melting of the composition and/or of the at least one silk protein, especially by means of a femtosecond laser.

8. Implantable hair according to any of Claims 1 to 7, wherein the production thereof comprises the partial removal of silk protein that has been applied to the hair, especially by means of plasma ablation, especially by means of a femtosecond laser.

9. Implantable hair according to any of Claims 1 to 8, wherein the implantable hair has been at least partly coated with a solid or pasty composition having a 3D surface structure in the nano- or micrometre range that differs from a largely cylindrical form.

10. Method of producing implantable hair according to at least one of Claims 1 to 9, wherein the method comprises the following steps:
(i) providing:
(A) hair that is not on the human or animal body, and
(B) a composition containing at least one silk protein; and
(ii) contacting at least an implantable section of the hair with the composition.

11. Method according to Claim 10, wherein the contacting step (ii) comprises:
(a) the dipping of the hair or a section thereof into the liquid or pasty composition;
(b) the coating or spraying of the hair or a section thereof with the liquid or pasty or solid composition;
(c) the sticking of the composition to the hair or a section thereof by means of a 3D shaping method, wherein a 3D surface structure in the nano- or micrometre range that differs from a largely cylindrical form is obtained;
(d) the sticking of the composition to the hair or a section thereof by means of the partial melting of the composition and/or of the at least one silk protein, especially by means of a femtosecond laser;
(e) the method comprises the further step of partly removing silk protein that has been applied to the hair, especially by means of plasma ablation, especially by means of a femtosecond laser,preferably affording a 3D surface structure in the nano- or micrometre range that differs from a largely cylindrical form.

12. Method according to either of Claims 10 or 11, wherein the method comprises the following steps:
(i) providing:
(A) hair that is not on the human or animal body, and
(B) a liquid or pasty or solid composition containing at least one silk protein;
(ii) contacting at least an implantable section of the hair with the liquid or pasty or solid composition; and
(iii) optionally drying the implantable hair obtained from step (ii);
and/or wherein the method comprises the following steps:
(i) providing:
(A) hair that is not on the human or animal body, and
(B) a largely solid or pasty composition containing at least one silk protein that may optionally constitute one or more fibres or an at least partly dried composition according to Claim 11;
(ii) contacting or maintaining contact of at least an implantable section of the hair with the composition, where steps (i) and (ii) may optionally also take place simultaneously,
(iii) sticking the composition to the hair or a section thereof by means of partial melting of the composition containing at least one silk protein, preferably by means of a pulse of light, especially by means of a femtosecond laser,
preferably affording a 3D surface structure in the nano- or micrometre range that differs from a largely cylindrical form.

13. Cosmetic use of implantable hair according to at least one of Claims 1 to 9 for implanting into human or animal skin, especially the human scalp.

14. Cosmetic method of implanting implantable hair according to at least one of Claims 1 to 9 into human or animal skin, especially the human scalp, comprising the following steps:
(i) providing hair according to at least one of Claims 1 to 9;
(ii) introducing the hair into the human or animal skin, especially the human scalp, by means of a needle.

## Revendications

1. Cheveu implantable, **caractérisé en ce qu'**il est revêtu, au moins sur une partie implantable, d'une composition entrant directement en contact avec le cheveu, qui contient au moins une protéine de soie.

2. Cheveu implantable selon la revendication 1, dans lequel ladite au moins une protéine de soie présente au moins 90 % d'homologie de séquence avec une protéine de soie d'araignée ou une protéine de soie d'insecte, de préférence dans lequel ladite au moins une protéine de soie présente au moins 90 % d'homologie de séquence avec une protéine de soie de l'un des genres *Araneus, Bombyx, Nephila, Antheraea, Arachnura, Caerostris, Argiope, Cyrtophora, Celaenia, Gasteracantha, Ordgarius, Neoscona, Zygiella, Parawixia, Neoscona, Dolophones, Aculeperia, Eriophora, Anepsion, Tegenaria, Heurodes, Cyclosa, Astracantha, Eriovixia, Nephilengys, Herennia, Acusilas, Neoscona, Poltys, Arkys, Poecilopachys Hyalophora, Samia, Attacus, Circula, Gonometa* et/ou *Anaphe,* plus de préférence dans lequel ladite au moins une protéine de soie présente au moins 90 % d'homologie de séquence avec une protéine de soie choisie parmi une espèce du groupe constitué par *Bombyx mori, Bombyx mandarina, Antheraea pernyi, Antheraea yamamai, Antheraea mylitta, Antheraea roylei, Antheraea proyeli, Antheraea paphia, Antheraea frithi, Antheraea assama, Araneus didenatus, Nephila clavipes, Araneus bicentenarius, Arachnura higginsi, Araneus circulissparsus, Araneus diadematus, Caerostris darwini, Argiope picta, Argiope trifasciata Nephila antipodiana, Cyrtophora beccarii, Celaenia excavata, Gasteracantha kuhlii, Argiope aurantia, Ordgarius furcatus, Ordgarius magnificus, Neoscona nautica, Neoscona rufofemorata, Zygiella calyptrata, Parawixia dehaani, Neoscona oxancensis, Gasteracantha cancriformis, Gasteracantha arcuata, Cyrtophora moluccensis, Cyrtophora parnasia, Dolophones conifera, Dolophones turrigera, Gasteracantha doriae, Gasteracantha mammosa, Cyrtophora exanthematica, Aculeperia ceropegia, Eriophora pustulosa, Anepsion depressium, Gasteracantha quadrispinosa, Eriophora transmarina, Araneus bicentenarius, Nephila maculata, Gasteracantha hasseltii, Tegenaria atrica, Heurodes turrita, Cyclosa insulana, Astracantha minax, Araneus mitificus, Eriovixia laglaisei, Cyclosa bifida, Nephilengys malabarensis, Argiope versicolor, Herennia ornatissima, Argiope aemula, Cyrtophora unicolor, Cyrtophora hirta, Argiope keyserlingi, Acusilas coccineus, Argiope argentata, Gasteracantha cancriformis, Neoscona domiciliorum, Argiope aetheria, Argiope Keyserlingi, Poltys illepidus, Arkys clavatus, Arkys lancearius, Poecilopachys australasia, Nephila clavipes, Nephila senegalensis, Nephila madagascariensis, Hyalophora cecropia, Samia Samia cynthia, Attacus atlas, Circula trifenestrata, Gonometa postica, Gonometa rufobrunnea, Anaphe panda* et/ou *Anaphe moloneyi,* en particulier dans lequel ladite au moins une protéine de soie présente au moins 90 % d'homologie de séquence avec une protéine de soie d'une espèce choisie dans le groupe constitué par *Bombyx mori, Antheraea pernyi, Araneus diadematus, Nephila clavipes, Araneus bicentenarius* et *Caerostris darwini.*

3. Cheveu implantable selon au moins l'une des revendications 1 ou 2, dans lequel ladite au moins une protéine de soie est **caractérisée en ce que** :
(a) elle est choisie dans le groupe constitué par la fibroïne, la séricine, la spidroïne 1 et la spidroïne 2 ; et/ou
(b) elle comprend au moins deux segments de séquence, qui présentent chacun l'une des séquences suivantes SEQ ID NO : 1 à 6 :
| | |
|---|---|
| GPGXX | (SEQ ID NO : 1), |
| GVPGX | (SEQ ID NO : 2), |
| GSGXX | (SEQ ID NO: 3), |
| GGYXX | (SEQ ID NO: 4), |
| PQQXX | (SEQ ID NO: 5), |
| GYGXX | (SEQ ID NO: 6), |
où chaque X peut être indépendamment n'importe quel acide aminé naturel, de préférence choisi dans le groupe constitué par V, G, A, S, Y, P et Q.

4. Cheveu implantable selon au moins l'une des revendications 1 à 3, dans lequel ladite au moins une partie implantable est une extrémité terminale implantable ou un pli implantable au milieu du cheveu ou une partie pliable au milieu du cheveu.

5. Cheveu implantable selon au moins l'une des revendications 1 à 4, ledit cheveu implantable étant **caractérisé en ce qu'**il :
(a) est un cheveu humain ;
(b) est un cheveu humain coupé, sans follicule pileux, sans racine pileuse et sans bulbe pileux ;
(c) est un cheveu synthétique ou un cheveu animal ; et/ou
(d) présentant sur toute sa longueur un diamètre moyen de 0,02 à 0,15 mm.

6. Cheveu implantable selon au moins l'une des revendications 1 à 5, ledit cheveu implantable étant **caractérisé par** au moins l'une des caractéristiques suivantes :
(1) le cheveu implantable ne présente pas d'épaississement sur la partie implantable, qui correspond à une circonférence maximale de plus de deux fois la circonférence moyenne du cheveu ;
(2) le cheveu implantable présente sur la partie implantable un épaississement artificiel formé par la composition, l'épaississement correspondant à une circonférence maximale autour du cheveu d'au moins deux fois la circonférence moyenne du cheveu ;
(3) le cheveu implantable présentant sur la partie implantable une structure d'ancrage artificielle qui peut être choisie en option dans le groupe constitué par la forme d'un cône, d'un cône tronqué, d'un cylindre épaissi, d'un champignon, d'une goutte, d'un ellipsoïde, d'un parallélépipède, d'une forme en spirale, d'une forme hélicoïdale, d'une forme annulaire, d'une sphère, d'une structure en nid d'abeilles, d'un enroulement partiel du cheveu avec une fibre ou un ruban et d'une combinaison de deux ou plusieurs de ceux-ci et, en option, des prolongements s'écartant de la structure du cheveu et/ou de la structure d'ancrage ;
(4) le cheveu implantable est incisé sur la partie implantable ;
(5) le cheveu implantable présente sur la partie implantable un follicule pileux ou une racine pileuse ou un bulbe pileux qui est revêtu de la composition ;
(6) deux ou plusieurs cheveux implantables sont réunis au niveau de leurs parties implantables dans une structure de revêtement ou d'ancrage ;
(7) le revêtement présentant des zones de solidité et/ou de stabilité biologique différentes, en particulier une solidité et/ou une stabilité biologique plus élevée à l'intérieur, près du cheveu, qu'à l'extérieur,
(8) le revêtement du cheveu implantable, comprenant au moins une protéine de soie, est conçu de telle sorte qu'après une implantation, la protéine de soie ou un produit de dégradation soit transporté vers le cheveu et/ou le cuir chevelu situés à l'extérieur du corps et y soigne le cheveu et/ou la peau.

7. Cheveu implantable selon au moins l'une des revendications 1 à 6, ledit cheveu implantable étant revêtu de la composition par façonnage 3D, en particulier par impression 3D,
en particulier sa fabrication comprenant la fusion au moins partielle de ladite composition et/ou de ladite au moins une protéine de soie, en particulier au moyen d'un laser femtoseconde.

8. Cheveu implantable selon l'une des revendications 1 à 7, dont la fabrication comprenant l'élimination partielle de la protéine de soie qui a été appliquée sur le cheveu, en particulier au moyen d'une ablation par plasma, en particulier au moyen d'un laser femtoseconde.

9. Cheveux implantables selon l'une des revendications 1 à 8, le cheveu implantable étant au moins partiellement revêtu d'une composition solide ou pâteuse qui présente une structure de surface tridimensionnelle de l'ordre du nanomètre ou du micromètre, qui s'écarte d'une forme largement cylindrique.

10. Procédé de fabrication de cheveu implantable selon au moins l'une des revendications 1 à 9, ledit procédé comprenant les étapes suivantes :
(i) mettre à disposition de :
(A) cheveu qui ne se trouve pas sur le corps humain ou animal, et
(B) une composition qui contient au moins une protéine de soie ; et
(ii) mettre en contact d'au moins une partie implantable du cheveu avec la composition.

11. Procédé selon la revendication 10, dans lequel l'étape (ii) de mettre en contact comprend :
(a) l'immersion du cheveu ou d'une partie de ceux-ci dans la composition liquide ou pâteuse ;
(b) l'application ou la pulvérisation de la composition liquide, pâteuse ou solide sur le cheveu ou une partie de ceux-ci ;
(c) l'adhérence de la composition sur le cheveu ou une partie de ceux-ci au moyen d'un procédé de façonnage 3D, dans lequel une structure de surface 3D de l'ordre du nanomètre ou du micromètre est obtenue, qui s'écarte d'une forme largement cylindrique ;
(d) l'adhérence de la composition sur le cheveu ou une partie de ceux-ci au moyen d'une fusion partielle de la composition et/ou de ladite au moins une protéine de soie, en particulier au moyen d'un laser femtoseconde ;
(e) le procédé comprend l'étape supplémentaire consistant à éliminer partiellement la protéine de soie qui a été appliquée sur le cheveu, en particulier au moyen d'une ablation au plasma, en particulier au moyen d'un laser femtoseconde, de préférence en obtenant une structure de surface 3D de l'ordre du nanomètre ou du micromètre qui s'écarte d'une forme largement cylindrique.

12. Procédé selon l'une des revendications 10 ou 11, ledit procédé comprenant les étapes suivantes :
(i) mettre à disposition de :
(A) cheveu qui ne se trouve pas sur le corps humain ou animal, et
(B) une composition liquide ou pâteuse ou solide contenant au moins une protéine de soie ;
(ii) mettre en contact d'au moins une partie implantable du cheveu avec la composition liquide ou pâteuse ou solide ; et
(iii) en option, séchage du cheveu implantables obtenus à l'étape (ii).
et/ou ledit procédé comprenant les étapes suivantes :
(i) mettre à disposition de :
(A) cheveu qui ne se trouve pas sur le corps humain ou animal, et
(B) une composition essentiellement solide ou pâteuse contenant au moins une protéine de soie, qui, en option, peut être une ou plusieurs fibres ou une composition au moins partiellement séchée selon la revendication 11 ;
(ii) mettre en contact ou maintenir en contact d'au moins une partie implantable du cheveu avec la composition, les étapes (i) et (ii) pouvant, en option, avoir lieu simultanément,
(iii) adhérer de la composition sur le cheveu ou une partie de ceux-ci au moyen d'une fusion partielle de la composition, qui contient au moins une protéine de soie, de préférence par une impulsion de lumière, en particulier d'un laser femtoseconde,
une structure de surface 3D de l'ordre du nanomètre ou du micromètre étant obtenue de préférence, qui s'écarte d'une forme largement cylindrique.

13. Utilisation cosmétique de cheveu implantable selon au moins l'une des revendications 1 à 9 pour l'implantation dans la peau humaine ou animale, en particulier le cuir chevelu humain.

14. Procédé cosmétique pour l'implantation de cheveu implantable selon au moins l'une des revendications 1 à 9 dans la peau humaine ou animale, en particulier le cuir chevelu humain, comprenant les étapes suivantes :
(i) mettre à disposition de cheveu selon au moins l'une des revendications 1 à 9 ;
(ii) introduire du cheveu dans la peau humaine ou animale, en particulier le cuir chevelu humain, au moyen d'une aiguille.
